(19) Europäisches Patentamt
European Patent Office
Office européen des brevets

(11) EP 2 138 158 B1

(12) **EUROPEAN PATENT SPECIFICATION**

(45) Date of publication and mention of the grant of the patent:
**30.09.2015 Bulletin 2015/40**

(21) Application number: **08720753.6**

(22) Date of filing: **19.02.2008**

(51) Int Cl.:
***A61K 8/64*** *(2006.01)* ***A61K 8/84*** *(2006.01)*
***A61K 8/97*** *(2006.01)* ***A61Q 9/04*** *(2006.01)*
***C07K 14/435*** *(2006.01)* ***C12N 15/09*** *(2006.01)*
***C12Q 1/02*** *(2006.01)* ***C12Q 1/68*** *(2006.01)*

(86) International application number:
**PCT/JP2008/052778**

(87) International publication number:
**WO 2008/102782 (28.08.2008 Gazette 2008/35)**

(54) **HAIR GROWTH-INHIBITING AGENT**
MITTEL ZUR UNTERDRÜCKUNG VON HAARWUCHS
AGENT INHIBITEUR DE CROISSANCE DES POILS

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **19.02.2007 JP 2007037844**

(43) Date of publication of application:
**30.12.2009 Bulletin 2009/53**

(73) Proprietor: **National Institute of Advanced Industrial Science and Technology**
**Tokyo 100-8921 (JP)**

(72) Inventors:
- **Imamura, Toru**
**Tsukuba-shi**
**Ibaraki 305-8566 (JP)**
- **Kimura, Miho**
**Tsukuba-shi**
**Ibaraki 305-8566 (JP)**
- **Kawano, Mitsuko**
**Tsukuba-shi**
**Ibaraki 305-8566 (JP)**
- **Tsujino, Nozomi**
**Tsukuba-shi**
**Ibaraki 305-0032 (JP)**
- **Kuramochi, Akiko**
**Tsukuba-shi**
**Ibaraki 305-8566 (JP)**
- **Oda, Yuko**
**Tsukuba-shi**
**Ibaraki 305-8566 (JP)**
- **Motomura, Kaori**
**Tsukuba-shi**
**Ibaraki 305-0044 (JP)**
- **Suzuki, Masashi**
**Tsukuba-shi**
**Ibaraki 305-8566 (JP)**
- **Asada, Masahiro**
**Tsukuba-shi**
**Ibaraki 305-8566 (JP)**
- **Kameyama, Azusa**
**Tsukuba-shi**
**Ibaraki 300-1241 (JP)**
- **Togayachi, Sumie**
**Ushiku-shi**
**Ibaraki 300-1206 (JP)**
- **Oka, Syuichi**
**Tsukuba-shi**
**Ibaraki 305-8566 (JP)**

(74) Representative: **Tuxworth, Pamela M.**
**J A Kemp**
**14 South Square**
**Gray's Inn**
**London WC1R 5JJ (GB)**

(56) References cited:
**WO-A1-2006/030693 US-A1- 2006 093 567**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **OTA YUTAKA ET AL: "Fibroblast growth factor 5 inhibits hair growth by blocking dermal papilla cell activation" BIOCHEMICAL AND BIOPHYSICAL RESEARCH COMMUNICATIONS, vol. 290, no. 1, 11 January 2002 (2002-01-11), pages 169-176, XP002587808 ISSN: 0006-291X**
- **KAWANO M. ET AL.: ' Comprehensive analysis of FGF and FGFR expression in skin: FGF18 is highly expressed in hair collicles and capable of inducing anagen from telogen stage hair follicles' J. INVEST. DERMATOL. vol. 124, no. 5, 2005, pages 877 - 885, XP003005530**
- **ZHANG X. ET AL.: 'Receptor specificity of the fibroblast growth factor family. The complete mammalian FGF family' J. BIOL. CHEM. vol. 281, no. 23, 2006, pages 15694 - 15700, XP008121923**


Remarks:

The file contains technical information submitted after the application was filed and not included in this specification

SEQUENCE LISTING

<110> National Institute of Advanced Industrial Science

<120> An Agent for growth suppression of hair

<130> 2007004261

<150> JP2007-37844
<151> 2007-02-19

<160> 30

<170> PatentIn version 3.1

<210> 1
<211> 549
<212> DNA
<213> human Full length(-signal peptide)FGF18

<400> 1

```
atggccgagg agaacgtgga cttccgcatc cacgtggaga accagacgcg ggctcgggac     60
gatgtgagcc gtaagcagct gcggctgtac cagctctaca gccggaccag tgggaaacac    120
atccaggtcc tgggccgcag gatcagtgcc cgcggcgagg atggggacaa gtatgcccag    180
ctcctagtgg agacagacac cttcggtagt caagtccgga tcaagggcaa ggagacggaa    240
ttctacctgt gcatgaaccg caaaggcaag ctcgtgggga agcccgatgg caccagcaag    300
gagtgtgtgt tcatcgagaa ggttctggag aacaactaca cggccctgat gtcggctaag    360
tactccggct ggtacgtggg cttcaccaag aaggggcggc cgcggaaggg ccccaagacc    420
cgggagaacc agcaggacgt gcatttcatg aagcgctacc ccaaggggca gccggagctt    480
cagaagccct tcaagtacac gacggtgacc aagaggtccc gtcggatccg gcccacacac    540
cctgcctga                                                            549
```

<210> 2
<211> 182
<212> PRT
<213> human Full length(-signal peptide)FGF18

<400> 2

```
Met Ala Glu Glu Asn Val Asp Phe Arg Ile His Val Glu Asn Gln Thr
1               5                   10                  15

Arg Ala Arg Asp Asp Val Ser Arg Lys Gln Leu Arg Leu Tyr Gln Leu
            20                  25                  30

Tyr Ser Arg Thr Ser Gly Lys His Ile Gln Val Leu Gly Arg Arg Ile
        35                  40                  45

Ser Ala Arg Gly Glu Asp Gly Asp Lys Tyr Ala Gln Leu Leu Val Glu
    50                  55                  60
```

```
Thr Asp Thr Phe Gly Ser Gln Val Arg Ile Lys Gly Lys Glu Thr Glu
65                  70                  75                  80

Phe Tyr Leu Cys Met Asn Arg Lys Gly Lys Leu Val Gly Lys Pro Asp
                85                  90                  95

Gly Thr Ser Lys Glu Cys Val Phe Ile Glu Lys Val Leu Glu Asn Asn
            100                 105                 110

Tyr Thr Ala Leu Met Ser Ala Lys Tyr Ser Gly Trp Tyr Val Gly Phe
        115                 120                 125

Thr Lys Lys Gly Arg Pro Arg Lys Gly Pro Lys Thr Arg Glu Asn Gln
    130                 135                 140

Gln Asp Val His Phe Met Lys Arg Tyr Pro Lys Gly Gln Pro Glu Leu
145                 150                 155                 160

Gln Lys Pro Phe Lys Tyr Thr Thr Val Thr Lys Arg Ser Arg Arg Ile
                165                 170                 175

Arg Pro Thr His Pro Ala
            180
```

<210> 3
<211> 549
<212> DNA
<213> mouse Full length(-signal peptide)FGF18

<400> 3

```
atggccgagg agaatgtgga cttccgcatc cacgtggaga accagacgcg ggctcgagat     60
gatgtgagtc ggaagcagct gcgcttgtac cagctctata gcaggaccag tgggaagcac    120
attcaagtcc tgggccgtag gatcagtgcc cgtggcgagg acggggacaa gtatgcccag    180
ctcctagtgg agacagatac cttcgggagt caagtccgga tcaagggcaa ggagacagaa    240
ttctacctgt gtatgaaccg aaaaggcaag ctcgtgggga agcctgatgg tactagcaag    300
gagtgcgtgt tcattgagaa ggttctggaa aacaactaca cggccctgat gtctgccaag    360
tactctggtt ggtatgtggg cttcaccaag aaggggcggc ctcgcaaggg tcccaagacc    420
cgcgagaacc agcaagatgt acacttcatg aagcgttacc ccaaggacaa ggccgagctg    480
cagaagccct tcaaatacac cacagtcacc aagcgatccc ggcggatccg ccccactcac    540
cccggctag                                                            549
```

<210> 4
<211> 537
<212> DNA

<213> Artificial

<220>
<223> truncation mutant FGF18(d4)

<400> 4

```
atggtggact tccgcatcca cgtggagaac cagacgcggg ctcgagatga tgtgagtcgg     60
aagcagctgc gcttgtacca gctctatagc aggaccagtg ggaagcacat tcaagtcctg    120
ggccgtagga tcagtgcccg tggcgaggac ggggacaagt atgcccagct cctagtggag    180
acagatacct tcgggagtca agtccggatc aagggcaagg agacagaatt ctacctgtgt    240
atgaaccgaa aaggcaagct cgtggggaag cctgatggta ctagcaagga gtgcgtgttc    300
attgagaagg ttctggaaaa caactacacg gccctgatgt ctgccaagta ctctggttgg    360
tatgtgggct tcaccaagaa ggggcggcct cgcaagggtc ccaagacccg cgagaaccag    420
caagatgtac acttcatgaa gcgttacccc aagggacagg ccgagctgca gaagcccttc    480
aaatacacca cagtcaccaa gcgatcccgg cggatccgcc ccactcaccc cggctag       537
```

<210> 5
<211> 513
<212> DNA
<213> Artificial

<220>
<223> truncation mutant FGF18(d12)

<400> 5

```
atgaaccaga cgcgggctcg agatgatgtg agtcggaagc agctgcgctt gtaccagctc     60
tatagcagga ccagtgggaa gcacattcaa gtcctgggcc gtaggatcag tgcccgtggc    120
gaggacgggg acaagtatgc ccagctccta gtggagacag ataccttcgg gagtcaagtc    180
cggatcaagg gcaaggagac agaattctac ctgtgtatga accgaaaagg caagctcgtg    240
gggaagcctg atggtactag caaggagtgc gtgttcattg agaaggttct ggaaaacaac    300
tacacggccc tgatgtctgc caagtactct ggttggtatg tgggcttcac caagaagggg    360
cggcctcgca agggtcccaa gacccgcgag aaccagcaag atgtacactt catgaagcgt    420
taccccaagg gacaggccga gctgcagaag cccttcaaat acaccacagt caccaagcga    480
tcccggcgga tccgccccac tcaccccggc tag                                 513
```

<210> 6
<211> 501
<212> DNA
<213> Artificial

<220>
<223> truncation mutant FGF18(d16)

<400> 6

```
atggctcgag atgatgtgag tcggaagcag ctgcgcttgt accagctcta tagcaggacc     60
agtgggaagc acattcaagt cctgggccgt aggatcagtg cccgtggcga ggacggggac    120
aagtatgccc agctcctagt ggagacagat accttcggga gtcaagtccg gatcaagggc    180
aaggagacag aattctacct gtgtatgaac cgaaaaggca agctcgtggg gaagcctgat    240
ggtactagca aggagtgcgt gttcattgag aaggttctgg aaaacaacta cacggccctg    300
atgtctgcca agtactctgg ttggtatgtg ggcttcacca agaaggggcg gcctcgcaag    360
ggtcccaaga cccgcgagaa ccagcaagat gtacacttca tgaagcgtta ccccaaggga    420
caggccgagc tgcagaagcc cttcaaatac accacagtca ccaagcgatc ccggcggatc    480
cgccccactc accccggcta g                                              501
```

<210> 7
<211> 495
<212> DNA
<213> Artificial

<220>
<223> truncation mutant FGF18(d18)

<400> 7

```
atggatgatg tgagtcggaa gcagctgcgc ttgtaccagc tctatagcag gaccagtggg     60
aagcacattc aagtcctggg ccgtaggatc agtgcccgtg gcgaggacgg ggacaagtat    120
gcccagctcc tagtggagac agataccttc gggagtcaag tccggatcaa gggcaaggag    180
acagaattct acctgtgtat gaaccgaaaa ggcaagctcg tggggaagcc tgatggtact    240
agcaaggagt gcgtgttcat tgagaaggtt ctggaaaaca actacacggc cctgatgtct    300
gccaagtact ctggttggta tgtgggcttc accaagaagg ggcggcctcg caagggtccc    360
aagacccgcg agaaccagca agatgtacac ttcatgaagc gttaccccaa gggacaggcc    420
gagctgcaga agcccttcaa atacaccaca gtcaccaagc gatcccggcg gatccgcccc    480
actcaccccg gctag                                                     495
```

<210> 8
<211> 483
<212> DNA
<213> Artificial

<220>
<223> truncation mutant FGF18(d22)

<400> 8

atgcggaagc agctgcgctt gtaccagctc tatagcagga ccagtgggaa gcacattcaa 60
gtcctgggcc gtaggatcag tgcccgtggc gaggacgggg acaagtatgc ccagctccta 120
gtggagacag ataccttcgg gagtcaagtc cggatcaagg gcaaggagac agaattctac 180
ctgtgtatga accgaaaagg caagctcgtg gggaagcctg atggtactag caaggagtgc 240
gtgttcattg agaaggttct ggaaaacaac tacacggccc tgatgtctgc caagtactct 300
ggttggtatg tgggcttcac caagaagggg cggcctcgca agggtcccaa gacccgcgag 360
aaccagcaag atgtacactt catgaagcgt taccccaagg gacaggccga gctgcagaag 420
cccttcaaat acaccacagt caccaagcga tcccggcgga tccgccccac tcaccccggc 480
tag 483

<210> 9
<211> 438
<212> DNA
<213> Artificial

<220>
<223> truncation mutant FGF18(d37)

<400> 9

atgaagcaca ttcaagtcct gggccgtagg atcagtgccc gtggcgagga cggggacaag 60
tatgcccagc tcctagtgga gacagatacc ttcgggagtc aagtccggat caagggcaag 120
gagacagaat tctacctgtg tatgaaccga aaaggcaagc tcgtggggaa gcctgatggt 180
actagcaagg agtgcgtgtt cattgagaag gttctggaaa acaactacac ggccctgatg 240
tctgccaagt actctggttg gtatgtgggc ttcaccaaga aggggcggcc tcgcaagggt 300
cccaagaccc gcgagaacca gcaagatgta cacttcatga agcgttaccc caagggacag 360
gccgagctgc agaagccctt caaatacacc acagtcacca agcgatcccg gcggatccgc 420
cccactcacc ccggctag 438

<210> 10
<211> 405
<212> DNA
<213> Artificial

<220>
<223> truncation mutant FGF18(d48)

<400> 10

```
atggcccgtg gcgaggacgg ggacaagtat gcccagctcc tagtggagac agataccttc      60
gggagtcaag tccggatcaa gggcaaggag acagaattct acctgtgtat gaaccgaaaa     120
ggcaagctcg tggggaagcc tgatggtact agcaaggagt gcgtgttcat tgagaaggtt     180
ctggaaaaca actacacggc cctgatgtct gccaagtact ctggttggta tgtgggcttc     240
accaagaagg ggcggcctcg caagggtccc aagacccgcg agaaccagca agatgtacac     300
ttcatgaagc gttaccccaa gggacaggcc gagctgcaga agcccttcaa atacaccaca     360
gtcaccaagc gatcccggcg gatccgcccc actcaccccg gctag                     405
```

<210> 11
<211> 348
<212> DNA
<213> Artificial

<220>
<223> truncation mutant FGF18(d67)

<400> 11

```
atggggagtc aagtccggat caagggcaag gagacagaat tctacctgtg tatgaaccga      60
aaaggcaagc tcgtggggaa gcctgatggt actagcaagg agtgcgtgtt cattgagaag     120
gttctggaaa acaactacac ggccctgatg tctgccaagt actctggttg gtatgtgggc     180
ttcaccaaga aggggcggcc tcgcaagggt cccaagaccc gcgagaacca gcaagatgta     240
cacttcatga agcgttaccc caagggacag gccgagctgc agaagccctt caaatacacc     300
acagtcacca agcgatcccg gcggatccgc cccactcacc ccggctag                  348
```

<210> 12
<211> 318
<212> DNA
<213> Artificial

<220>
<223> truncation mutant FGF18(d77)

<400> 12

```
atgacagaat tctacctgtg tatgaaccga aaaggcaagc tcgtggggaa gcctgatggt      60
actagcaagg agtgcgtgtt cattgagaag gttctggaaa acaactacac ggccctgatg     120
tctgccaagt actctggttg gtatgtgggc ttcaccaaga aggggcggcc tcgcaagggt     180
cccaagaccc gcgagaacca gcaagatgta cacttcatga agcgttaccc caagggacag     240
gccgagctgc agaagccctt caaatacacc acagtcacca agcgatcccg gcggatccgc     300
cccactcacc ccggctag                                                   318
```

<210> 13
<211> 264
<212> DNA
<213> Artificial

<220>
<223> truncation mutant FGF18(d95)

<400> 13

```
atgggtacta gcaaggagtg cgtgttcatt gagaaggttc tggaaaacaa ctacacggcc     60
ctgatgtctg ccaagtactc tggttggtat gtgggcttca ccaagaaggg gcggcctcgc    120
aagggtccca agacccgcga gaaccagcaa gatgtacact tcatgaagcg ttaccccaag    180
ggacaggccg agctgcagaa gcccttcaaa tacaccacag tcaccaagcg atcccggcgg    240
atccgcccca ctcaccccgg ctag                                           264
```

<210> 14
<211> 182
<212> PRT
<213> mouse Full length(-signal peptide) FGF18

<400> 14

```
Met Ala Glu Glu Asn Val Asp Phe Arg Ile His Val Glu Asn Gln Thr
1               5                   10                  15

Arg Ala Arg Asp Asp Val Ser Arg Lys Gln Leu Arg Leu Tyr Gln Leu
            20                  25                  30

Tyr Ser Arg Thr Ser Gly Lys His Ile Gln Val Leu Gly Arg Arg Ile
        35                  40                  45

Ser Ala Arg Gly Glu Asp Gly Asp Lys Tyr Ala Gln Leu Leu Val Glu
    50                  55                  60

Thr Asp Thr Phe Gly Ser Gln Val Arg Ile Lys Gly Lys Glu Thr Glu
65                  70                  75                  80

Phe Tyr Leu Cys Met Asn Arg Lys Gly Lys Leu Val Gly Lys Pro Asp
                85                  90                  95

Gly Thr Ser Lys Glu Cys Val Phe Ile Glu Lys Val Leu Glu Asn Asn
            100                 105                 110

Tyr Thr Ala Leu Met Ser Ala Lys Tyr Ser Gly Trp Tyr Val Gly Phe
        115                 120                 125

Thr Lys Lys Gly Arg Pro Arg Lys Gly Pro Lys Thr Arg Glu Asn Gln
    130                 135                 140

Gln Asp Val His Phe Met Lys Arg Tyr Pro Lys Gly Gln Ala Glu Leu
145                 150                 155                 160

Gln Lys Pro Phe Lys Tyr Thr Thr Val Thr Lys Arg Ser Arg Arg Ile
                165                 170                 175

Arg Pro Thr His Pro Gly
            180
```

<210> 15
<211> 178
<212> PRT
<213> Artificial

<220>
<223> truncation mutant FGF18(d4)

<400> 15

Met Val Asp Phe Arg Ile His Val Glu Asn Gln Thr Arg Ala Arg Asp
1 5 10 15

Asp Val Ser Arg Lys Gln Leu Arg Leu Tyr Gln Leu Tyr Ser Arg Thr
20 25 30

Ser Gly Lys His Ile Gln Val Leu Gly Arg Arg Ile Ser Ala Arg Gly
35 40 45

Glu Asp Gly Asp Lys Tyr Ala Gln Leu Leu Val Glu Thr Asp Thr Phe
50 55 60

Gly Ser Gln Val Arg Ile Lys Gly Lys Glu Thr Glu Phe Tyr Leu Cys
65 70 75 80

Met Asn Arg Lys Gly Lys Leu Val Gly Lys Pro Asp Gly Thr Ser Lys
85 90 95

Glu Cys Val Phe Ile Glu Lys Val Leu Glu Asn Asn Tyr Thr Ala Leu
100 105 110

Met Ser Ala Lys Tyr Ser Gly Trp Tyr Val Gly Phe Thr Lys Lys Gly
115 120 125

Arg Pro Arg Lys Gly Pro Lys Thr Arg Glu Asn Gln Gln Asp Val His
130 135 140

Phe Met Lys Arg Tyr Pro Lys Gly Gln Ala Glu Leu Gln Lys Pro Phe
145 150 155 160

Lys Tyr Thr Thr Val Thr Lys Arg Ser Arg Arg Ile Arg Pro Thr His
165 170 175

Pro Gly

<210> 16
<211> 170
<212> PRT
<213> Artificial

<220>
<223> truncation mutant FGF18 (d12)

<400> 16

Met Asn Gln Thr Arg Ala Arg Asp Asp Val Ser Arg Lys Gln Leu Arg
1 5 10 15

Leu Tyr Gln Leu Tyr Ser Arg Thr Ser Gly Lys His Ile Gln Val Leu
20 25 30

Gly Arg Arg Ile Ser Ala Arg Gly Glu Asp Gly Asp Lys Tyr Ala Gln
35 40 45

Leu Leu Val Glu Thr Asp Thr Phe Gly Ser Gln Val Arg Ile Lys Gly
50 55 60

Lys Glu Thr Glu Phe Tyr Leu Cys Met Asn Arg Lys Gly Lys Leu Val
65 70 75 80

Gly Lys Pro Asp Gly Thr Ser Lys Glu Cys Val Phe Ile Glu Lys Val
85 90 95

Leu Glu Asn Asn Tyr Thr Ala Leu Met Ser Ala Lys Tyr Ser Gly Trp
100 105 110

Tyr Val Gly Phe Thr Lys Lys Gly Arg Pro Arg Lys Gly Pro Lys Thr
115 120 125

Arg Glu Asn Gln Gln Asp Val His Phe Met Lys Arg Tyr Pro Lys Gly
130 135 140

Gln Ala Glu Leu Gln Lys Pro Phe Lys Tyr Thr Thr Val Thr Lys Arg
145 150 155 160

Ser Arg Arg Ile Arg Pro Thr His Pro Gly
165 170

<210> 17
<211> 166
<212> PRT
<213> Artificial

<220>
<223> truncation mutant FGF18(d16)

<400> 17

Met Ala Arg Asp Asp Val Ser Arg Lys Gln Leu Arg Leu Tyr Gln Leu
1 5 10 15

Tyr Ser Arg Thr Ser Gly Lys His Ile Gln Val Leu Gly Arg Arg Ile
20 25 30

Ser Ala Arg Gly Glu Asp Gly Asp Lys Tyr Ala Gln Leu Leu Val Glu
35 40 45

Thr Asp Thr Phe Gly Ser Gln Val Arg Ile Lys Gly Lys Glu Thr Glu
50 55 60

Phe Tyr Leu Cys Met Asn Arg Lys Gly Lys Leu Val Gly Lys Pro Asp
65 70 75 80

Gly Thr Ser Lys Glu Cys Val Phe Ile Glu Lys Val Leu Glu Asn Asn
85 90 95

Tyr Thr Ala Leu Met Ser Ala Lys Tyr Ser Gly Trp Tyr Val Gly Phe
100 105 110

Thr Lys Lys Gly Arg Pro Arg Lys Gly Pro Lys Thr Arg Glu Asn Gln
115 120 125

Gln Asp Val His Phe Met Lys Arg Tyr Pro Lys Gly Gln Ala Glu Leu
130 135 140

Gln Lys Pro Phe Lys Tyr Thr Thr Val Thr Lys Arg Ser Arg Arg Ile
145 150 155 160

Arg Pro Thr His Pro Gly
165

<210> 18
<211> 164
<212> PRT
<213> Artificial

<220>
<223> truncation mutant FGF18(d18)

<400> 18

Met Asp Asp Val Ser Arg Lys Gln Leu Arg Leu Tyr Gln Leu Tyr Ser
1 5 10 15

Arg Thr Ser Gly Lys His Ile Gln Val Leu Gly Arg Arg Ile Ser Ala
20 25 30

Arg Gly Glu Asp Gly Asp Lys Tyr Ala Gln Leu Leu Val Glu Thr Asp
35 40 45

Thr Phe Gly Ser Gln Val Arg Ile Lys Gly Lys Glu Thr Glu Phe Tyr
50 55 60

Leu Cys Met Asn Arg Lys Gly Lys Leu Val Gly Lys Pro Asp Gly Thr
65 70 75 80

Ser Lys Glu Cys Val Phe Ile Glu Lys Val Leu Glu Asn Asn Tyr Thr

85 90 95

Ala Leu Met Ser Ala Lys Tyr Ser Gly Trp Tyr Val Gly Phe Thr Lys
100 105 110

Lys Gly Arg Pro Arg Lys Gly Pro Lys Thr Arg Glu Asn Gln Gln Asp
115 120 125

Val His Phe Met Lys Arg Tyr Pro Lys Gly Gln Ala Glu Leu Gln Lys
130 135 140

Pro Phe Lys Tyr Thr Thr Val Thr Lys Arg Ser Arg Arg Ile Arg Pro
145 150 155 160

Thr His Pro Gly

<210> 19
<211> 160
<212> PRT
<213> Artificial

<220>
<223> truncation mutant FGF18(d22)

<400> 19

```
Met Arg Lys Gln Leu Arg Leu Tyr Gln Leu Tyr Ser Arg Thr Ser Gly
1               5                   10                  15

Lys His Ile Gln Val Leu Gly Arg Arg Ile Ser Ala Arg Gly Glu Asp
            20                  25                  30

Gly Asp Lys Tyr Ala Gln Leu Leu Val Glu Thr Asp Thr Phe Gly Ser
        35                  40                  45

Gln Val Arg Ile Lys Gly Lys Glu Thr Glu Phe Tyr Leu Cys Met Asn
    50                  55                  60

Arg Lys Gly Lys Leu Val Gly Lys Pro Asp Gly Thr Ser Lys Glu Cys
65                  70                  75                  80

Val Phe Ile Glu Lys Val Leu Glu Asn Asn Tyr Thr Ala Leu Met Ser
                85                  90                  95

Ala Lys Tyr Ser Gly Trp Tyr Val Gly Phe Thr Lys Lys Gly Arg Pro
            100                 105                 110

Arg Lys Gly Pro Lys Thr Arg Glu Asn Gln Gln Asp Val His Phe Met
        115                 120                 125

Lys Arg Tyr Pro Lys Gly Gln Ala Glu Leu Gln Lys Pro Phe Lys Tyr
    130                 135                 140

Thr Thr Val Thr Lys Arg Ser Arg Arg Ile Arg Pro Thr His Pro Gly
145                 150                 155                 160
```

<210> 20
<211> 145
<212> PRT
<213> Artificial

<220>
<223> truncation mutant FGF18(d37)

<400> 20

```
Met Lys His Ile Gln Val Leu Gly Arg Arg Ile Ser Ala Arg Gly Glu
1               5                   10                  15

Asp Gly Asp Lys Tyr Ala Gln Leu Leu Val Glu Thr Asp Thr Phe Gly
            20                  25                  30

Ser Gln Val Arg Ile Lys Gly Lys Glu Thr Glu Phe Tyr Leu Cys Met
        35                  40                  45

Asn Arg Lys Gly Lys Leu Val Gly Lys Pro Asp Gly Thr Ser Lys Glu
    50                  55                  60

Cys Val Phe Ile Glu Lys Val Leu Glu Asn Asn Tyr Thr Ala Leu Met
65                  70                  75                  80

Ser Ala Lys Tyr Ser Gly Trp Tyr Val Gly Phe Thr Lys Lys Gly Arg
                85                  90                  95

Pro Arg Lys Gly Pro Lys Thr Arg Glu Asn Gln Gln Asp Val His Phe
            100                 105                 110

Met Lys Arg Tyr Pro Lys Gly Gln Ala Glu Leu Gln Lys Pro Phe Lys
        115                 120                 125

Tyr Thr Thr Val Thr Lys Arg Ser Arg Arg Ile Arg Pro Thr His Pro
    130                 135                 140

Gly
145
```

<210> 21
<211> 134
<212> PRT
<213> Artificial

<220>
<223> truncation mutant FGF18(d48)

<400> 21

Met Ala Arg Gly Glu Asp Gly Asp Lys Tyr Ala Gln Leu Leu Val Glu
1 5 10 15

Thr Asp Thr Phe Gly Ser Gln Val Arg Ile Lys Gly Lys Glu Thr Glu
20 25 30

Phe Tyr Leu Cys Met Asn Arg Lys Gly Lys Leu Val Gly Lys Pro Asp
35 40 45

Gly Thr Ser Lys Glu Cys Val Phe Ile Glu Lys Val Leu Glu Asn Asn
50 55 60

Tyr Thr Ala Leu Met Ser Ala Lys Tyr Ser Gly Trp Tyr Val Gly Phe
65 70 75 80

Thr Lys Lys Gly Arg Pro Arg Lys Gly Pro Lys Thr Arg Glu Asn Gln
85 90 95

Gln Asp Val His Phe Met Lys Arg Tyr Pro Lys Gly Gln Ala Glu Leu
100 105 110

Gln Lys Pro Phe Lys Tyr Thr Thr Val Thr Lys Arg Ser Arg Arg Ile
115 120 125

Arg Pro Thr His Pro Gly
130

<210> 22
<211> 115
<212> PRT
<213> Artificial

<220>
<223> truncation mutant FGF18(d67)

<400> 22

Met Gly Ser Gln Val Arg Ile Lys Gly Lys Glu Thr Glu Phe Tyr Leu
1 5 10 15

Cys Met Asn Arg Lys Gly Lys Leu Val Gly Lys Pro Asp Gly Thr Ser
20 25 30

Lys Glu Cys Val Phe Ile Glu Lys Val Leu Glu Asn Asn Tyr Thr Ala
35 40 45

Leu Met Ser Ala Lys Tyr Ser Gly Trp Tyr Val Gly Phe Thr Lys Lys
50 55 60

Gly Arg Pro Arg Lys Gly Pro Lys Thr Arg Glu Asn Gln Gln Asp Val
65 70 75 80

His Phe Met Lys Arg Tyr Pro Lys Gly Gln Ala Glu Leu Gln Lys Pro
85 90 95

Phe Lys Tyr Thr Thr Val Thr Lys Arg Ser Arg Arg Ile Arg Pro Thr
100 105 110

His Pro Gly
115

<210> 23
<211> 105
<212> PRT
<213> Artificial

<220>
<223> truncation mutant FGF18(d77)

<400> 23

Met Thr Glu Phe Tyr Leu Cys Met Asn Arg Lys Gly Lys Leu Val Gly
1 5 10 15

Lys Pro Asp Gly Thr Ser Lys Glu Cys Val Phe Ile Glu Lys Val Leu
20 25 30

Glu Asn Asn Tyr Thr Ala Leu Met Ser Ala Lys Tyr Ser Gly Trp Tyr
35 40 45

Val Gly Phe Thr Lys Lys Gly Arg Pro Arg Lys Gly Pro Lys Thr Arg
50 55 60

Glu Asn Gln Gln Asp Val His Phe Met Lys Arg Tyr Pro Lys Gly Gln
65 70 75 80

Ala Glu Leu Gln Lys Pro Phe Lys Tyr Thr Thr Val Thr Lys Arg Ser
85 90 95

Arg Arg Ile Arg Pro Thr His Pro Gly
100 105

<210> 24
<211> 87
<212> PRT
<213> Artificial

<220>

<223> truncation mutant FGF18(d95)

<400> 24

**Met Gly Thr Ser Lys Glu Cys Val Phe Ile Glu Lys Val Leu Glu Asn**
**1 5 10 15**

**Asn Tyr Thr Ala Leu Met Ser Ala Lys Tyr Ser Gly Trp Tyr Val Gly**
**20 25 30**

**Phe Thr Lys Lys Gly Arg Pro Arg Lys Gly Pro Lys Thr Arg Glu Asn**
**35 40 45**

**Gln Gln Asp Val His Phe Met Lys Arg Tyr Pro Lys Gly Gln Ala Glu**
**50 55 60**

**Leu Gln Lys Pro Phe Lys Tyr Thr Thr Val Thr Lys Arg Ser Arg Arg**
**65 70 75 80**

**Ile Arg Pro Thr His Pro Gly**
**85**

<210> 25
<211> 525
<212> DNA
<213> Artificial

<220>
<223> truncation mutant FGF18(dc8)

<400> 25

**atggccgagg agaatgtgga cttccgcatc cacgtggaga accagacgcg ggctcgagat 60**

**gatgtgagtc ggaagcagct gcgcttgtac cagctctata gcaggaccag tgggaagcac 120**

**attcaagtcc tgggccgtag gatcagtgcc cgtggcgagg acggggacaa gtatgcccag 180**

**ctcctagtgg agacagatac cttcgggagt caagtccgga tcaagggcaa ggagacagaa 240**

**ttctacctgt gtatgaaccg aaaaggcaag ctcgtgggga agcctgatgg tactagcaag 300**

**gagtgcgtgt tcattgagaa ggttctggaa aacaactaca cggccctgat gtctgccaag 360**

**tactctggtt ggtatgtggg cttcaccaag aaggggcggc ctcgcaaggg tcccaagacc 420**

**cgcgagaacc agcaagatgt acacttcatg aagcgttacc ccaagggaca ggccgagctg 480**

**cagaagccct tcaaatacac cacagtcacc aagcgatccc ggtag 525**

<210> 26
<211> 501
<212> DNA
<213> Artificial

<220>
<223> truncation mutant FGF18(dc15)

<400> 26

atggccgagg agaatgtgga cttccgcatc cacgtggaga accagacgcg ggctcgagat 60
gatgtgagtc ggaagcagct gcgcttgtac cagctctata gcaggaccag tgggaagcac 120
attcaagtcc tgggccgtag gatcagtgcc cgtggcgagg acggggacaa gtatgcccag 180
ctcctagtgg agacagatac cttcgggagt caagtccgga tcaagggcaa ggagacagaa 240
ttctacctgt gtatgaaccg aaaaggcaag ctcgtgggga agcctgatgg tactagcaag 300
gagtgcgtgt tcattgagaa ggttctggaa aacaactaca cggccctgat gtctgccaag 360
tactctggtt ggtatgtggg cttcaccaag aaggggcggc ctcgcaaggg tcccaagacc 420
cgcgagaacc agcaagatgt acacttcatg aagcgttacc ccaagggaca ggccgagctg 480
cagaagccct tcaaatacta g 501

<210> 27
<211> 474
<212> DNA
<213> Artificial

<220>
<223> truncation mutant FGF18(dc25)

<400> 27

atggccgagg agaatgtgga cttccgcatc cacgtggaga accagacgcg ggctcgagat 60
gatgtgagtc ggaagcagct gcgcttgtac cagctctata gcaggaccag tgggaagcac 120
attcaagtcc tgggccgtag gatcagtgcc cgtggcgagg acggggacaa gtatgcccag 180
ctcctagtgg agacagatac cttcgggagt caagtccgga tcaagggcaa ggagacagaa 240
ttctacctgt gtatgaaccg aaaaggcaag ctcgtgggga agcctgatgg tactagcaag 300
gagtgcgtgt tcattgagaa ggttctggaa aacaactaca cggccctgat gtctgccaag 360
tactctggtt ggtatgtggg cttcaccaag aaggggcggc ctcgcaaggg tcccaagacc 420
cgcgagaacc agcaagatgt acacttcatg aagcgttacc ccaagggaca gtag 474

<210> 28
<211> 174
<212> PRT
<213> Artificial

<220>
<223> truncation mutant FGF18(dc8)

<400> 28

Met Ala Glu Glu Asn Val Asp Phe Arg Ile His Val Glu Asn Gln Thr
1 5 10 15

Arg Ala Arg Asp Asp Val Ser Arg Lys Gln Leu Arg Leu Tyr Gln Leu
20 25 30

Tyr Ser Arg Thr Ser Gly Lys His Ile Gln Val Leu Gly Arg Arg Ile
35 40 45

Ser Ala Arg Gly Glu Asp Gly Asp Lys Tyr Ala Gln Leu Leu Val Glu
50 55 60

Thr Asp Thr Phe Gly Ser Gln Val Arg Ile Lys Gly Lys Glu Thr Glu
65 70 75 80

Phe Tyr Leu Cys Met Asn Arg Lys Gly Lys Leu Val Gly Lys Pro Asp
85 90 95

Gly Thr Ser Lys Glu Cys Val Phe Ile Glu Lys Val Leu Glu Asn Asn
100 105 110

Tyr Thr Ala Leu Met Ser Ala Lys Tyr Ser Gly Trp Tyr Val Gly Phe
115 120 125

Thr Lys Lys Gly Arg Pro Arg Lys Gly Pro Lys Thr Arg Glu Asn Gln
130 135 140

Gln Asp Val His Phe Met Lys Arg Tyr Pro Lys Gly Gln Ala Glu Leu
145 150 155 160

Gln Lys Pro Phe Lys Tyr Thr Thr Val Thr Lys Arg Ser Arg
165 170

<210> 29
<211> 167
<212> PRT
<213> Artificial

<220>
<223> truncation mutant FGF18(dc15)

<400> 29

Met Ala Glu Glu Asn Val Asp Phe Arg Ile His Val Glu Asn Gln Thr
1 5 10 15

Arg Ala Arg Asp Asp Val Ser Arg Lys Gln Leu Arg Leu Tyr Gln Leu
20 25 30

Tyr Ser Arg Thr Ser Gly Lys His Ile Gln Val Leu Gly Arg Arg Ile
35 40 45

Ser Ala Arg Gly Glu Asp Gly Asp Lys Tyr Ala Gln Leu Leu Val Glu
50 55 60

Thr Asp Thr Phe Gly Ser Gln Val Arg Ile Lys Gly Lys Glu Thr Glu
65 70 75 80

Phe Tyr Leu Cys Met Asn Arg Lys Gly Lys Leu Val Gly Lys Pro Asp
85 90 95

Gly Thr Ser Lys Glu Cys Val Phe Ile Glu Lys Val Leu Glu Asn Asn
100 105 110

Tyr Thr Ala Leu Met Ser Ala Lys Tyr Ser Gly Trp Tyr Val Gly Phe
115 120 125

Thr Lys Lys Gly Arg Pro Arg Lys Gly Pro Lys Thr Arg Glu Asn Gln
130 135 140

Gln Asp Val His Phe Met Lys Arg Tyr Pro Lys Gly Gln Ala Glu Leu
145 150 155 160

Gln Lys Pro Phe Lys Tyr Thr
165

<210> 30
<211> 157
<212> PRT
<213> Artificial

<220>
<223> truncation mutant FGF18(dc25)

<400> 30

```
Met Ala Glu Glu Asn Val Asp Phe Arg Ile His Val Glu Asn Gln Thr
1               5                   10                  15

Arg Ala Arg Asp Asp Val Ser Arg Lys Gln Leu Arg Leu Tyr Gln Leu
            20                  25                  30

Tyr Ser Arg Thr Ser Gly Lys His Ile Gln Val Leu Gly Arg Arg Ile
        35                  40                  45

Ser Ala Arg Gly Glu Asp Gly Asp Lys Tyr Ala Gln Leu Leu Val Glu
    50                  55                  60

Thr Asp Thr Phe Gly Ser Gln Val Arg Ile Lys Gly Lys Glu Thr Glu
65                  70                  75                  80

Phe Tyr Leu Cys Met Asn Arg Lys Gly Lys Leu Val Gly Lys Pro Asp

                85                  90                  95

Gly Thr Ser Lys Glu Cys Val Phe Ile Glu Lys Val Leu Glu Asn Asn
            100                 105                 110

Tyr Thr Ala Leu Met Ser Ala Lys Tyr Ser Gly Trp Tyr Val Gly Phe
        115                 120                 125

Thr Lys Lys Gly Arg Pro Arg Lys Gly Pro Lys Thr Arg Glu Asn Gln
    130                 135                 140

Gln Asp Val His Phe Met Lys Arg Tyr Pro Lys Gly Gln
145                 150                 155
```

## Description

TECHNICAL FIELD

The present invention relates to methods and uses for a hair growth-inhibiting agent or a hair removing agent both comprising a substance which inhibits the growth of hair follicles (synonymous with hair roots), and a method of screening for such substances.

BACKGROUND ART

It is known that a variety of polypeptide growth factors, including various members of the fibroblast growth factor (hereinafter, referred to as "FGF") family, are expressed in skin tissue. In both mouse and human, FGFs are encoded by 22 distinct genes (Non-Patent Document 1). Among them, FGF1, FGF2, FGF5, FGF7, FGF10, FGF13 and FGF22 are reported to be expressed in dermal cells and hair follicular cells to regulate hair growth and skin regeneration (see Non-Patent Documents 2-17 and Patent Document 1).

Non-Patent Document 2-17 and other documents suggest that FGF plays an important role in the growth and differentiation of dermal cells. However, it is still unknown as to how the FGF group is involved in the effect of promoting or inhibiting the growth of hair follicles and the resultant hair growth promoting effect or hair growth inhibiting effect.

Under the above-described circumstances, the present inventors found that single administration of FGF18 to mouse skin with hair folliclles in telogen phase (resting phase) induces hair regrowth, and reported that FGF18 is a substance which induces onset of anagen phase (growth phase) in follicles to result in promotion of hair growth (Non-Patent Document 19 and Patent Document 2).

Non-Patent Document 1: Ornitz DM, Itoh N: Fibroblast growth factors. Genome Biol2: REVIE WS3005, 2001

Non-Patent Document 2: du Cros DL: Fibroblast growth factor and epidermal growth factor in hair development. J Invest Dermatol 101: 106S-113S. 1993

Non-Patent Document 3: du Cros DL, Isaacs K, Moore GP: Distribution of acidic and basicfibr oblast growth factorsin ovine skin during follicle morphogenesis. J Cell Sci 105: 667-674,1993

Non-Patent Document 4: Herbert JM, Rosenquist T, Gotz J, Martin GR: FGF5 as a regulator of the hair growth cycle: Evidence from targeted and spontaneous mutations. Cell78: 1017-1025, 1994

Non-Patent Document 5: Danilenko DM, Ring BD, Yanagihara D, Benson W, Wiemann B, StamesCO, Pierce GF: Keratinocyte growth factor is an important endogenous mediator of hair follicle growth, development, and differentiation. American J Pathol147: 145-154, 1995

Non-Patent Document 6: Marchese C, Chedid M, Dirsch OR, et al: Modulation of keratinocyte growth factor and its receptor in reepithelializing human skin. J Exp Med182: 1369-1376, 1995

Non-Patent Document 7: Guo L, Degenstein L, Fuchs E: keratinocyte growth factor is require dfor hair development but not for wound healing. Genes Dev 10: 165-175, 1996

Non-Patent Document 8: Rosenquist TA, Martin GR: Fibroblast growth factor signalling in the hair growth cycle: Expression of the fibroblast growth factor receptor and ligand genes in the murine hair follicle. Developmental Dynamics 205:379-386, 1996

Non-Patent Document 9: Petho-Schramm A, Muller HJ, Paus R: FGF5 and the murine hair cycle. Arch Dermatol Res 288: 264-266, 1996

Non-Patent Document 10: Mitsui S, Ohuchi A, Hotta M, Tsuboi R, Ogawa H: Genes for a range of growth factors and cyclin-dependent kinase inhibitors are expressed by isolated human hair follicles. Br J Dermatol 137:693-698, 1997

Non-Patent Document 11: Ortega S, Ittmann M, Tsang SH, Ehrlich M, Basilico C: Neuronaldefects and delayed wound healing in mice lacking fibroblast growth factor-2. Proc Natl Acad Sci USA 95: 5672-5677, 1998

Non-Patent Document 12: Suzuki S, Kato T, Takimoto H, et al: Localization of rat FGF-5 protein in skin macrophage-like cells and FGF-5S protein in hair follicle: Possible involvement of two Fgf-5 gene products in hair growth cycleregulation. J Invest Dermatol 111: 963-972, 1998

Non-Patent Document 13: Suzuki S, Ota Y, Ozawa K, Imamura T: Dual-mode regulation of hair growth cycle by two Fgf-5 gene products. J Invest Dermatol 114: 456-463, 2000

Non-Patent Document 14: Nakatake Y, Hoshikawa M, Asaki T, Kassai Y, Itoh N: Identification of a novel fibroblast growth factor, FGF-22, preferentially expressed in the inner root sheath of the hair follicle. Biochem Biophys Acta 1517: 460-463, 2001

Non-Patent Document 15: Stenn KS, Paus R: Controls of hair follicle cycling. Physiol Rev81: 449-494, 2001

Non-Patent Document 16: Beyer TA, Werner S, Dickson C, Grose R: Fibroblast growth factor 22 and its potential role during skin development and repair. Exp Cell Res287: 228-236 2003

Non-Patent Document 17: kawano M, Suzuki S, Suzuki M, Oki J, Imamura T: Bulge- and basallayer-specific expression of fibroblast growth factor 13(FHF-2) in mouse skin. J Invest Dermatol 122: 1084-1090, 2004

Non-Patent Document 18: Suzuki S, Ota Y, Ozawa K, Imamura T: Dual-mode regulation of hair growth cycle by two Fgf-5 gene products. J Invest Dermatol 114: 456-463, 2000

Non-Patent Document 19: Kawano M, Komi-Kuramochi A, Asada M, Suzuki M, Oki J, Jiang J, Imamura T: Comprehensive analysis of FGF and FGFR expression in skin: FGF18 is highly expressed in hair follicles and capable of inducing anagen from telogenstage hair follicles. J Invest Dermatol. 2005, 124(5): 877-885

Non-Patent Document 20: ZhangX, Ibrahimi OA, Olsen SK, Umemori H, Mohammadi M, Ornitz DM. Receptor Specificity of the Fibroblast Growth Factor Family: THE COMPLETE MAMMALIAN FGFFAMILY J Biol Chem 2006, 281(23):15694-15700

Patent Document 1: Japanese Unexamined Patent Publication No. Hei 4-224522

Patent Document 2: Japanese Unexamined Patent Publication No. 2006-83082

DISCLOSURE OF THE INVENTION

PROBLEM FOR SOLUTION BY THE INVENTION

Many people are having trouble with unwanted hair. To cope with this problem, removal of the hair shaft is often carried out using chemical substances or by means of this physical methods. However, the effect is soon lost as a result of natural follicle growth. It is an object of the present invention to elucidate the mode of action of endogenous factors that regulate hair growth, to determine an endogenous factor that inhibits hair growth, and to provide a hair growth-inhibiting agent or a hair removing agent using the endogenous factor. Further, it is another object of the present invention to screen for substances that promote the activity or expression of the endogenous factor and substances that have an effect similar to the effect of the endogenous factor, and to provide those substances which have a hair growth-inhibiting effect similar to the effect of the endogenous factor.

It is still another object of the present invention to provide a screening method for the above-described purpose.

MEANS TO SOLVE THE PROBLEM

As described above, it was already confirmed that single administration of FGF18 to skin with hair follicles in telogen phase promotes hair growth after several weeks of reaction period. Therefore, FGF18 was believed to be a substance that has a hair growth promoting effect.

However, the present inventors have obtained an unexpected, surprising finding when FGF18 was administered to mouse dorsal skin subcutaneously once a day for 8 days after compulsive induction of anagen phase in follicles of the dorsal skin in telogen phase by depilating, so that FGF18 was allowed to persist in hair follicles continuously for observing the state of growth of hair.

Briefly, hair growth progressed well and follicle enlargement occurred at day 9 in the control group which received phosphate-buffered physiological saline under similar conditions whereas hair growth was markedly inhibited the FGF18 administered group.

Further, the present inventors performed a detailed functional analysis of FGF18. As a result, it was found that the expression level of a growth factor VEGF (which is considered important for hair follicle growth) decreased when dermal papilla cells (which are believed to be a control tower for hair follicle growth) were cultured in the presence of FGF18.

The present inventors have achieved the present invention based on this unexpected, novel finding that FGF18 (which was believed to be a hair growth promoting substance) "acts to inhibit hair growth" when administered continuously.

Considering that FGF18 is an endogenous factor present in hair follicles by nature, a substance that has an activity similar to that of FGF18 on hair follicles, a substance that promotes the activity of FGF18 *per se* or a substance that activates the expression of *FGF18* can clearly be used as a hair growth-inhibiting agent or hair removing agent.

Substances which activate the expression of *FGF18* may be screened for by monitoring whether a test substance promotes the expression of *FGF18* or not in a cultured animal cell or an experimental animal.

Substances which promote the activity of FGF18 *per se* may be screened for by bringing a test substance together with FGF18 into contact with an FGF receptor on cell surfaces and monitoring whether the test substance promotes the activity of FGF18 *per se* on the FGF receptor.

Specifically, those substances may be obtained by a screening method comprising the following steps (a) to (c):

(a) compulsively expressing at least one FGF receptor gene selected from *FGFR1c, FGFR2c, FGFR3c* and *FGFR4* on the surface of a cell by means of genetic engineering and culturing the cell,
(b) bringing, together with FGF18, a test substance into contact with the cell system obtained in step (a) having the FGF receptor on cell surfaces; and
(c) selecting those test substances which exhibited a higher cell growth promoting activity in step (b) than when FGF18 was allowed to act singly.

Further, on the assumption that a substance having an activity similar to that of FGF18 would have a similar hair growth-inhibiting effect, the present inventors focused on the reactivity of FGF18 on FGF receptors present in hair follicle cells. Since FGF18 reacts with FGFR1c, FGFR2c, FGFR3c and FGFR4 among the FGF receptor subclasses, a screening system for FGF18-like active substances was developed based on the above reactivity (Non-Patent Document 20).

As a result of extensive searches into substances derived from naturally occurring plants and other natural products using FGF4 receptor as the screening system, several natural product-derived substances which activate FGF4 receptor were found. Thus, the present invention relating to FGF18-like active substances has also been achieved.

The present invention includes the following inventions.

(1) A method of non-therapeutic hair growth inhibition or non-therapeutic hair removal comprising administering FGF18 and/or *Digenea simplex* extract and/or an expression vector carrying an FGF18-encoding DNA to hair follicles which are in the anagen phase.
(2) The method according to (1) wherein said FGF18 and/or *Digenea simplex* extract and/or expression vector carrying an FGF18-encoding DNA is used in combination with another hair growth-inhibiting agent or hair removing agent.
(3) The method according to (1) or (2) wherein said FGF 18 and/or extract and/or expression vector carrying an FGF18-encoding DNA is administered so that the FGF18 and/or *Digenea simplex* extract and/or expression vector carrying an FGF18-encoding DNA remains in hair follicles at a concentration of at least 1% of the initial concentration for a period of at least 1 day.
(4) The method according to any one of (1) to (3) wherein said anagen phase has been induced by depilating.
(5) A method of non-therapeutic hair growth inhibition or non-therapeutic hair removal comprising the steps of:

(i) inducing anagen phase in hair follicles; and
(ii) administering to said hair follicles FGF18 and/or *Digenea simplex* extract and/or expression vector carrying an FGF18-encoding DNA.

(6) The method according to any one of (1) to (5), wherein:

(a) the expression vector carrying an FGF18 encoding DNA integrated thereinto is being administered so that the FGF18 is expressed in hair follicles continuously; or
(b) said FGF18 is a full-length peptide of the amino acid sequence as shown in SEQ ID NO: 2 or a partial peptide there of having FGF18-like activity.

(7) FGF18 and/or *Digenea simplex* extract and/or expression vector carrying an FGF18-encoding DNA for use in a method of therapeutic hair growth inhibition or therapeutic hair removal wherein said FGF18 and/or extract and/or is administered to hair follicles which are in anagen phase.
(8) The FGF 18 and/or extract and/or expression vector carrying an FGF 18-encoding DNA for use according to (7) wherein said FGF18 and/or *Digenea simplex* extract and/or expression vector carrying an FGF18-encoding DNA is used in combination with another hair growth-inhibiting agent or hair removing agent.
(9) The FGF18 and/or extract and/or an expression vector carrying an FGF18-encoding DNA for use according to (7) or (8) wherein said FGF18 and/or *Digenea simplex* extract and/or expression vector carrying an FGF18-encoding DNA is administered so that the FGF18 and/or *Digenea simplex* extract and/or expression vector carrying an FGF18-encoding DNA remains in hair follicles at a concentration of at least 1% of the initial concentration for a period of at least 1 day.
(10) The FGF 18 and/or extract and/or expression vector carrying an FGF18-encoding DNA for use according to any one of (7) to (9) wherein said anagen phase has been induced by depilating.
(11) The FGF18 and/or extract and/or expression vector carrying an FGF18-encoding DNA for use according to any one of (7) to (10) wherein:

(a) the expression vector carrying an FGF18-encoding DNA integrated thereinto is being administered so that the FGF18 is expressed in hair follicles continuously; or
(b) said FGF18 is a full-length peptide of the amino acid sequence as shown in SEQ ID NO: 2 or a partial peptide there of having FGF18-like activity.

(12) A method of screening for a substance that promotes the hair growth inhibiting activity of FGF18 as defined in any one of (1) to (6) to thereby obtain candidates for the hair growth-inhibiting agent or hair removing agent from test substances, said method comprising the following steps (a) to (d):

(a) preparing a non-human experimental animal or a cultured animal cell capable of expressing *FGF18* to an observable extent,
(b) bringing a test substance into contact with the experimental animal, or the cultured animal cell
(c) monitoring the expression of *FGF18* in the experimental animal or the cultured animal cell after step (b), and
(d) selecting those test substances which have a function of promoting the expression of *FGF18*.

(13) The method according to (12), wherein the expression of *FGF18* is monitored in step (c) by extracting mRNA from the experimental animal or the cultured animal cell after step (b) and then analyzing the mRNA level of expressed *FGF18*; and those test substances which have a function of promoting the expression of *FGF18* are selected in step (d) by selecting systems that exhibited higher levels of *FGF18* mRNA than when the test substance was not allowed to act.

EFFECT OF THE INVENTION

According to the present invention, it is possible to inhibit hair growth or remove hair by using or imitating an endogenous factor which inhibits the growth of hair follicles.
Further, according to the screening method of the present invention, it is possible to screen for substances which are effective as hair growth-inhibiting agent or hair removing agent.

BRIEF DESCRIPTION OF THE DRAWINGS

Fig. 1. is a graph showing that the mRNA copy number of a hair follicle growth factor released by dermal papilla cells is suppressed at a low level when FGF18 is present continuously.
Fig. 2 is a diagram demonstrating *in vivo* that it is possible to inhibit the growth of hair follicles by continuous administration of FGF18. In this Figure, A is a photomicrograph of a skin section from a control mouse that received PBS; and B is a photomicrograph of a skin section from a mouse that received FGF18.
Fig. 3 is a graph demonstrating that N-terminal truncated FGF18 partial peptides have an FGF receptor stimulating effect. (DNA synthesis levels when FGF receptor expressing cells are stimulated with about 100 ng/ml of samples are shown.) In this Figure, R1c, R2c, R3c and R4 represent FGFR1c expressing cell, FGFR2c expressing cell, FGFR3c expressing cell and FGFR4 expressing cell, respectively. Columns 1, 9, 16 and 26 are control columns without addition of samples; columns 2, 10, 17 and 27 received sample (d4); columns 3, 18 and 28 received sample (d12); columns 19 and 29 received sample (d16); columns 4, 20 and 30 received sample (d18); columns 5, 11,21 and 31 received sample (d22); columns 6, 22 and 32 received sample (d37); column 12 received sample (d48); columns 13, 23 and 33 received sample (d67); columns 7, 14, 24 and 34 received sample (d77); and columns 8, 15 and 95 received sample (d95).

Fig. 4 is graphs demonstrating that C-terminal truncated FGF18 partial peptides have an FGF receptor stimulating effect. (DNA synthesis levels when FGF receptor expressing cells are stimulated with various concentrations of samples are shown.)

Fig. 5 R4/Ba/F3 Cell Growth Promoting Effect of *Digenea simplex* Extract

Proliferation rates of R4/Ba/F3 cells when *Digenea simplex* extract was added at 0.083%, 0.83% and 8.3% in the presence and absence of FGF18 are shown.

Fig. 6 *In vivo* Analysis of *Digenea simplex* Extract

The time course of the state of hair regrowth in mouse dorsal skin is shown after *Digenea simplex* extract was administered twice subcutaneously to a dorsal site of C3H/HeN mouse in telogen phase of the hair cycle.

BEST MODE FOR CARRYING OUT THE INVENTION

Hereinbelow, the present invention will be described in detail.

By applying the present invention, it is possible to provide methods and uses for a hair growth-inhibiting agent and hair removing agent wherein the agent is FGF18 and/or *Digenea simplex* extract and/or an expression vector carrying an FGF18-encoding DNA. These hair growth-inhibiting agent and hair removing agent share the common feature of utilizing the effect that FGF 18 of consistently high concentrations inhibits the growth of hair follicles (sometimes called "hair roots").

The hair follicle is an organ that produces hair. The hair follicle growth cycle consists of a growth phase (anagen), a recessing phase (catagen) and a resting phase (telogen) which follows the recessing phase. After the resting phase, the growth phase begins. Generally, in mouse experimental systems, anagen phase is a period from day 1 to day 19 after depilation; and catagen phase is a period from day 20 to day 21 after depilation. It is also known that the hair follicle cycle enters telogen phase at day 21-22 after depilation. During the anagen phase, the growth (elongation) of new hair is activated. Simultaneously, the growth of hair follicles is activated inside the skin, and the bottom part of hair follicles reaches the vicinity of the skin bottom. On the other hand, during the telogen phase, hair follicles stay shallow in the skin in small sizes. Further, the thickness of the skin is completely different between anagen phase and telogen phase. In mice with a colored body hair, melanin is synthesized at the beginning of the anagen phase and a blue skin is visible. Therefore, it is also possible to evaluate the progress of hair follicle growth cycle by observing the blue color from the outside of the skin. Further, when the skin is dissected during the anagen phase and observed from the reverse side, the reverse side of the skin can be seen black because hair follicles with abundant melanin are standing in lines at a high density. To the contrary, during the telogen phase, the reverse side of the skin can be seen remaining white. For example, in mouse experimental systems, the entire dorsal hair of 7 to 8 week-old mice is in telogen phase; by depilating the grown hair, the hair cycle is synchronized and anagen phase begins.

1. FGF18

FGF18 is synthesized in the cytoplasm of FGF18 producing cells as a polypeptide of 207 amino acids in human and mouse. When this polypeptide is secreted to the outside of cells, its N-terminal signal peptide is cleaved to generate a secreted polypeptide of 181 amino acids which exerts a physiological action. It has been confirmed that FGF18 reacts with at least four out of the seven FGF receptor subclasses (FGFR1c, FGFR1b, FGFR2c, FGFR2b, FGFR3c, FGFR3b and FGFR4) and they are FGFR1c, FGFR2c, FGFR3c and FGFR4 (Non-Patent Document 20).

Examples of the FGF 18 contained in the novel hair growth-promoting agent, hair regrowth-promoting agent or therapeutic for alopecia of the present invention include a human-derived FGF18 consisting of the amino acid sequence as shown in SEQ ID NO: 2. FGF18 polypeptides which may be used in the present invention are not limited to human-derived polypeptides but include, for example, polypeptides derived from other mammals. Specific examples of other mammals include, but are not limited to, mouse, rat, chicken, turkey, cattle, pig, sheep and rabbit. For example, it is possible to isolate a gene encoding FGF 18 from a non-human mammal by designing a probe based on the nucleotide sequence of a human-derived FGF18 as shown in SEQ ID NO: 1 and using the probe according to conventional methods.

The nucleotide sequence and the amino acid sequence of secretion signal sequence-deleted human-derived FGF18 are shown in SEQ ID NOS: 1 and 2, respectively. The nucleotide sequence and the amino acid sequence of secretion signal sequence-deleted mouse-derived FGF18 are shown in SEQ ID NOS: 3 and 14, respectively. As comparison of the amino acid sequences as shown in SEQ ID NOS: 2 and 14 reveals, FGF18 polypeptides have a very high homology in mammals. Thus, it is understood that the function of FGF 18 is almost the same among mammals.

In the present invention, "the number of amino acids deleted at the N-terminal" means the number of deleted amino acids excluding the methionine residue introduced for initiation of translation in recombinant protein production. Those proteins which consist of the amino acid sequence as shown in SEQ ID NO: 2 or 14, with one or several amino acids being deleted, substituted or added, and which have a hair follicle growth-inhibiting effect are included in the FGF18 of the present invention. It should be noted that "several amino acids" means, for example, 2 to 37 amino acids, preferably 2 to 22 amino acids, more preferably 2 to 18 amino acids, even more preferably 2 to 12 amino acids, and most preferably 2 to 4 amino acids.

The region spanning from positions 32 to 151 of the amino acid sequence as shown in SEQ ID NO: 14 is believed to be a core domain common to various FGF18 polypeptides; it has been observed that this domain binds to receptors and heparin (Non-Patent Document 1). Therefore, partial peptides comprising positions 32 to 151 of the amino acid sequence as shown in SEQ ID NO: 14 or mutant peptides with one or several amino acids deleted, substituted or added in a region other than the above-described region from positions 32 to 151 may be used as the FGF18 of the present invention as long as those peptides exert the hair follicle growth-inhibiting effect possessed by FGF18.

Actually, according to the results of experiments (Fig. 3) where the effect on FGF18 activity of deleting 4 to 95 amino acids (excluding the methionine residue) from the N-terminal of SEQ ID NO: 14 (d4-d95) was determined, even d95 possesses FGF18-like FGFR3c stimulating activity although it is weak.

Therefore, the amino acids at positions 1 to 95 may be entirely deleted from the N-terminus of SEQ ID NO: 14 (excluding the methionine residue) and yet the resulting polypeptide can be used as FGF18 in the present invention. Preferably, a partial peptide with the amino acids at positions 1 to 77 deleted from the N-terminus is used. More preferably a partial peptide with the amino acids at positions 1 to 37 deleted from the N-terminus, still more preferably a partial peptide with the amino acids at positions 1 to 22 deleted from the N-terminus, even more preferably a partial peptide with the amino acids at positions 1 to 12 deleted from the N-terminus, and most preferably a partial peptide with the amino acids at positions 1 to 4 deleted from the N-terminus are used. Further, a part of the N-terminal sequence from positions 1 to 95 may be substituted; preferably, a partial peptide comprising the amino acid sequence from positions 24 to 182 of SEQ ID NO: 14 may be used; and more preferably, a partial peptide comprising the amino acid sequence from positions 6 to 182 of SEQ ID NO: 14 may be used.

With respect to the C-terminal side, according to the results of experiments where the effect on FGF18 activity of deleting C-terminal 8 to 25 amino acids of SEQ ID NO: 14 (dc8-dc25) was determined, even dc25 possesses FGF18-like FGFR2c, FGFR3c and FGFR4 stimulating activity strongly (Fig. 4). Therefore, the amino acids at positions 1 to 25 may be entirely deleted from the C-terminus of SEQ ID NO: 14 and yet the resulting polypeptide can be used as FGF18 in the present invention.

As described above, the term "FGF18" as used herein includes not only full-length polypeptides of FGF18 derived from mammals such as human and mouse, but also FGF18 partial peptides and FGF 18 mutant peptides having FGF18-like activity.

2. FGF18-Like Active Substances, FGF18 Activating Substances and Screening Methods for Such Substances

[1] FGF18-Like Active Substances

The term "FGF18-like active substance" means a substance which binds to a receptor similar to the receptor that FGF 18 binds to and which activates an intracellular signal transduction system similar to the system that FGF18 activates. Since FGF18 reacts with at least the four FGF receptor subclasses FGFR1c, FGFR2c, FGFR3c and FGFR4 among the seven FGF receptor subclasses as described above, the "FGF18-like active substance" of the present invention means a substance which binds to any of the receptors FGFR1c, FGFR2c, FGFR3c and FGFR4 on the surface of a cell and activates the intracellular signal transduction system of the cell in the same manner as FGF18. (However, among those FGF18-like active substances, FGF18 partial peptides and FGF18 mutant peptides are included in the "FGF18" in the present invention.) In order to specifically examine whether or not a substance activates the intracellular signal transduction system of a cell that possesses FGF receptor on its surface, the amounts of intracellular second messengers (such as Ca ion or cAMP) may be measured. Typically, this activation is confirmed when proliferation of an FGF receptor-expressing cell has been observed after administering the substance to the cell or transferring the gene of the substance into the cell and expressing the gene therein.

In particular, FGF18 has a strong effect to activate the intracellular signal transduction system in FGFR3c-expressing cell and FGFR4-expressing cell among receptor-expressing cells. Therefore, it is also possible to select FGF18-like active substances as substances having a strong activity upon FGFR3c-expressing cell or FGFR4-expressing cell. Further, it is also possible to select FGF18-like active substances as substances whose cell growth promoting activity upon FGFR3c-expressing cell and FGFR4-expressing cell is markedly higher (at least twice, preferably at least three times) than their cell growth promoting activity upon FGFR1c-expressing cell and FGFR2c-expressing cell at the same concentration.

[2] Screening Methods for FGF18-Like Active Substances

Screening for those substances which potentially have a function of hair growth inhibition, hair removal or the like can be performed by examining whether or not a test substance has the above-described FGF 18-like activity.
Specifically, a screening method comprising the following steps (a) to (c) may be used.

(a) compulsively expressing at least one FGF receptor gene selected from *FGFR1c, FGFR2c, FGFR3c* and *FGFR4* on the surface of a cell by means of genetic engineering and culturing the cell,
(b) bringing a test substance into contact with the cell system obtained in step (a) having the FGF receptor on cell surfaces; and
(c) selecting those test substances which exhibited FGF18-like cell growth promoting activity in step (b).

Alternatively, a screening method comprising the following steps (a') to (c') may be used.

(a') compulsively expressing four FGF receptor genes, *FGFR1c, FGFR2c*, *FGFR3c* and *FGFR4,* on respective cell surfaces by means of genetic engineering and culturing the four cells,
(b') bringing a test substance into contact with the four cell systems obtained in step (a') having the FGF receptors on cell surfaces; and
(c') selecting those test substances which, similar to EGF18, exhibit markedly higher cell growth promoting activity on the FGFR3c-expressing cell and the FGFR4-expressing cell than on the FGFR1c-expressing cell and the FGFR2c-expressing cell at the same concentration.

As the FGF receptor gene, at least one of the FGF receptor genes *FGFR1c, FGFR2c, FGFR3c* and *FGFR4* is used; it has been confirmed that FGF 18 binds to these receptors and that FGF18 exerts cell growth effect upon cells having these receptors on their surfaces. Preferably, *FGFR3c* or *FGFR4* is used. To bring a test substance into contact with a cell, it is typically directly added to the cell culture broth, but in a particular case where the test substance is a protein, a gene encoding the test substance can be transferred into an FGF receptor-expressing cell.
The cell to be used for compulsive expression of an FGF receptor on its surface may be any cell as long as it can be cultured. Preferably, mouse IL-3-dependent Ba/F3 cell strain is used.
The parent cell with no FGF receptor gene transferred thereinto may be used in a control test. It is preferable to provide a step of performing the similar operation as in step (b) using a test substance to confirm that the test substance does not cause cell growth promoting activity in such parent cells.

[3] Substances that Promote the Activity of FGF18

Substances even if they can not be said to be FGF18-like active substance as defined in [1] above, may foster the hair follicle growth-inhibiting effect of FGF18 by, for example, promoting or enhancing the binding of FGF18 to receptors; since these substances have hair growth-inhibiting effect or hair removing effect, they are referred to as "substances that promote the activity of FGF18" in the present invention. These substances may be used either alone or in combination with FGF18, etc. as a hair growth-inhibiting agent or hair removing agent.

[4] Screening Method for Substances that Promote the Activity of FGF18

Substances that promote the activity of FGF 18 may be screened for by bringing, together with FGF18, a test substance into contact with cell surface FGF receptors and then monitoring whether the activity of FGF 18 *per se* on FGF receptors has been promoted.
Specifically, substances that promote the activity of FGF 18 may be obtained by a screening method comprising the following steps (a) to (c):

(a) compulsively expressing at least one FGF receptor gene selected from *FGFR1c, FGFR2c, FGFR3c* and *FGFR4* on the surface of a cell by means of genetic engineering and culturing the cell,
(b) bringing, together with FGF 18, a test substance into contact with the cell system obtained in step (a) having the FGF receptor on cell surfaces; and
(c) selecting those cell systems which exhibited a higher cell growth promoting activity in step (b) than when FGF 18 alone was allowed to act singly.

Those test substances which increased the cell growth promoting activity of FGF18 on cells expressing FGF receptors (such as FGFR3c or FGFR4) in this screening method are believed to be substances that promote the activity of FGF 18 in hair follicle cells, so they can be judged to have a potential function of hair growth inhibition or hair removal.

Accordingly, the FGF18 activity promoting substance screened through those steps is a promising candidate for a hair growth-inhibiting agent or hair removing agent. As a criterion for screening, those substances which increased the proliferation capacity of FGF receptor expressing cells by 5% or more, preferably by 10% or more, more preferably by 20% or more, may be selected.

3. Substances that Promote the Expression of FGF18 and Screening Method for Such Substances

Since FGF18 is an endogenous factor present in hair follicles, a substance that promotes the transcription and translation of *FGF18* in hair follicle cells (i.e., substance that activates the expression of *FGF18*) is also capable of increasing the concentration of FGF18 in hair follicle cells to thereby induce the hair growth inhibiting activity of FGF18. Thus, such a substance has a hair growth inhibiting or hair removing effect.

Such substances that activate the expression of *FGF18* may be screened for by monitoring whether or not a test substance promotes the expression of *FGF18* in cultured animal cells (such as skin-derived cells) or experimental animals capable of expressing FGF18 to an observable extent.

Specifically, first, a test substance is brought into contact with or administered to an experimental animal, or a test substance is brought into contact with a cultured animal cell. Experimental animals refer to non-human animals such as mouse, rat, chicken, turkey, cattle, pig, sheep and rabbit. Examples of test substances include, but are not limited to, plant extracts, peptides, proteins, nonpeptidic compounds, low molecular weight compounds, synthetic compounds, fermentation products, cell extracts and animal tissue extracts. These substances may be either novel substances or known substances. To bring it into contact with a cell or animal, the test substance is typically directly added to the cell culture broth or administered to the experimental animal but in a particular case where the test substance is a protein, a gene encoding the test substance can be transferred into an FGF receptor-expressing cell.

Subsequently, the expression of *FGF18* in the cultured animal cell or experimental animal is monitored. The expression of *FGF18* in the cultured animal cell or experimental animal may be monitored, for example, by analysis with conventional methods such as ELISA using FGF18 antibody or by analyzing the *FGF18* mRNA level in the cell or experimental animal by quantitative reverse transcription PCR or Northern blotting.

If, as a result of analysis by any of the above-listed methods, the expression level of *FGF18* in the cultured animal cell or experimental animal cultured in the presence of a test substance is found to be greater than that level in the animal cell cultured in the absence of the test substance, the test substance may be judged to have a potential function of hair growth inhibition or hair rem oval. Specifically, if the mRNA level is at least 20%, preferably at least 50%, more preferably at least 80%, still more preferably at least 100% greater than in the case where the test substance was not allowed to act, the latter can positively be regarded as a substance that promotes the expression of FGF18. The expression levels of *FGF18* mRNA in cultured keratinocytes, cultured dermal cells or cultured dermal papilla cells vary considerably depending on culture conditions or the type of the cells, so they may be measured individually by the above-mentioned methods or the like and 1.2-fold or greater increases in measured values may be used as a criterion for screening.

The FGF18 expression promoting substance screened through the above-described steps may be used either alone or in combination with FGF18, etc. as a hair growth-inhibiting agent or a hair removing agent.

4. Hair Growth Inhibiting Agent and Hair Removing Agent

In the present invention, it is necessary to administer the above-described FGF18, *Digenea simplex* extract or expression vector carrying an FGF18-encoding DNA (hereinafter, referred to as "FGF18 or the like") in such a manner that FGF18 or the like is allowed to persist in hair follicles continuously. The expression "persist continuously" means such a state that FGF18 or the like remains in hair follicles at a concentration of at least 1%, preferably at least 5%, more preferably at least 10% of the initial concentration, for a period of at least 1 day, preferably 4 days, more preferably 8 days. In order to achieve such a state, a method may be used in which FGF18 or the like is gradually absorbed from the skin using implants or patches. Alternatively, FGF18 or the like may be administered repeatedly depending on necessity, for example, at intervals of 1 to 3 days or at least twice a day.

With respect to specific dosage forms, FGF 18 or the like is formulated either alone or in combination into preparations adapted for skin application (e.g., solutions, creams, ointments, gels, lotions, shampoos, aerosols or patches) and is supplied as a hair growth inhibiting agent or a hair removing agent.

In particular, the hair growth inhibiting agent or hair removing agent is administered in the form of a pharmaceutical composition comprising FGF18 or the like together with a pharmacologically acceptable carrier adapted for local application. The hair growth inhibiting agent or hair removing agent comprising FGF18 or the like contains an active compound in a pharmacologically acceptable carrier usually at about 0.01 to about 100 $\mu g/day/cm^2$, preferably about 0.1 to about 10 $\mu g/day/cm^2$. This means that the concentration of FGF18 or the like is usually about 0.01 to about 100

μg/day/cm$^2$, preferably about 0.1 to about 10 μg/day/cm$^2$ in a pharmacologically acceptable carrier.

Further, the hair growth inhibiting agent or hair removing agent may comprise other hair growth inhibiting agents or hair removing agents well known in the art. Substances which increase or enhance the hair growth inhibiting or hair removing effect of FGF18 or the like are not particularly limited.

Further, the hair growth inhibiting agent or hair removing agent may comprise compounds or drugs well known in the art that exhibit hair growth inhibiting activity or hair removing activity. Other hair growth inhibiting agents or hair removing agents are not particularly limited.

Further, the pharmacologically acceptable carrier adapted for local application is not particularly limited. Specific examples include, but are not limited to, ointments such as hydrophilic vaseline or polyethylene glycol ointment; pastes such as gum (e.g., xanthan gum); solutions such as alcoholic, aqueous or buffer solution; gels such as aluminum hydroxide or sodium alginate gel; albumins such as human or animal albumin; collagens such as human or animal collagen; celluloses such as alkyl cellulose, hydroxyalkyl cellulose or alkylhydroxyalkyl cellulose; methyl cellulose, hydroxyethyl cellulose, carboxymethyl cellulose, hydroxypropylmethyl cellulose and hydroxypropyl cellulose; polymers such as Pluronic™ polyol as exemplified by Pluronic F-127; tetronics such as Tetronic 1508; and alginates such as sodium alginate.

The hair growth inhibiting agent or hair removing agent of the present invention may be one that comprises, as an active ingredient, an expression vector carrying an FGF18-encoding DNA as described in the above subsection "1. FGF18" (i.e., a DNA encoding the full-length polypeptide of FGF18 derived from a mammal such as human, or a partial peptide thereof or a mutant peptide thereof, both having FGF18-like activity). This means that the hair growth inhibiting agent or hair removing agent of the present invention may be used in gene therapy using the above expression vector. The expression vector is not particularly limited, although it has sequences (such as promoter) to drive the expression of FGF18 or the like in animal cells. Examples of useful expression vectors include, but are not limited to, plasmid vectors and virus vectors.

More specifically, in gene therapy, the FGF18-encoding DNA as described in the above subsection "1. FGF18" is integrated in, for example, a virus vector. Then, an avirulent virus comprising the resultant recombinant virus vector is transfected into patients. FGF18 is produced in patients' bodies. This FGF18 is capable of inhibiting the growth of hair follicles.

As a method of introducing the hair growth inhibiting agent or hair removing agent for gene therapy into cells, both a gene transfer method using a virus vector and a non-viral gene transfer method [Nikkei Science, 1994 April issue, pp. 20-45; Experimental Medicine Extra Issue 12(15)(1994); Experimental Medicine Separate Volume "Basic Techniques for Gene Therapy", Yodo-sha (1996)] may be used.

As examples of gene transfer method using a virus vector, methods may be mentioned in which a DNA encoding TR4 or mutant TR4 is integrated in DNA or RNA viruses such as retrovirus, adenovirus, adeno-associated virus, herpes virus, vaccinia virus, poxvirus, poliovirus and sinbis virus. Among these, methods using retrovirus, adenovirus, adeno-associated virus or vaccinia virus are especially preferred.

Examples of non-viral gene-transfer methods include a method in which an expression plasmid is directly administered into muscle (DNA vaccine method), the liposome method, lipofection, microinjection, the calcium phosphate method, electroporation and so on. The DNA vaccine method and the liposome method are especially preferred.

In order to allow the hair growth inhibiting agent or hair removing agent for gene therapy to actually work as a pharmaceutical, an *in vivo* method (in which DNA is introduced into the body directly) or an *ex vivo* method (in which a certain type of cell is taken out of the human body; DNA is introduced into the cell *ex vivo*; and then the cell is returned to the body) [Nikkei Science, 1994 April issue, pp. 20-45; The Pharmaceuticals Monthly, 36(1), 23-48(1994); Experimental Medicine Extra Issue 12(15) (1994)]may be used.

For example, when the agent for gene therapy is administered by an *in vivo* method, it may be administered through an appropriate route, such as intravenous, intra-arterial, subcutaneous, intradermal or intramuscular route, depending on the type of disease and the severity of condition. When the agent for gene therapy is administered by an *in vivo* method, the agent is generally administered in the form of injections to which conventional carriers may be added, if necessary. Alternatively, when the agent for gene therapy is administered in the form of liposomes or membrane-fused liposomes (e.g., Sendai virus-liposome), liposome preparations such as suspensions, frozen preparations, centrifuged/concentrated/frozen preparations, or the like may be used.

5. *Digenea simplex* Extract

One of the FGF18-like active substances obtained by the screening method described in the above subsection 2. [2] is *Digenea simplex* extract. This extract has been confirmed to have a hair regrowth inhibiting effect.

The raw material *Digenea simplex* belongs to the family *Rhodomelaceae,* the order *Ceramiales* in the class *Florideophyceae.* It is also called "Makuri".

In obtaining extract from *Digenea simplex,* the entire part of this plant may be used; individual parts may be used either independently or in an appropriate combination. Further, the plant may be used whether it is in a dry or non-dry state.

For obtaining an extract to be used in the present invention from *Digenea simplex,* the raw material may be shredded or crushed and then extracted with an appropriate solvent by conventional extraction methods. The solvent used is not particularly limited. For example, water or anhydrous or hydrous organic solvents may be enumerated. Examples of anhydrous or hydrous organic solvents include one or more substances selected from the group consisting of monohydric alcohols, polyhydric alcohols or derivatives thereof, ketones, esters, ethers, petroleum ether, aliphatic hydrocarbons, halogen compounds and aromatic hydrocarbons. Specific examples of the solvent include, but are not limited to, water, methanol, ethanol, butanol, acetone and ethyl acetate ester. They may be used either alone or in combination. Among these, use of water or a monohydric alcohol such as methanol or ethanol is especially preferable. For extracting purposes, the amounts of the above-listed solvents are not particularly limited. The amount of the solvent may be 0.1-1000 times, preferably 1-100 times, more preferably 2-50 times by weight the amount of the raw material *Digenea simplex.*

The extraction method using the above-listed solvents may be performed according to conventional procedures. For example, as regards the extraction temperature, extraction may be performed at around room temperature or at a temperature around the boiling point of the solvent used. As regards the extraction operation, a dried and crushed or a simply crushed *Digenea simplex* may be soaked in a solvent at room temperature for 1-30 days or may be extracted under reflux at a temperature around the boiling point of the solvent.

As will be described later in Example 4, the *Digenea simplex* extract was searched for as one of those substances which have FGF18-like cell growth promoting activity by the screening method of the present invention using a cell upon whose surface FGFR4 (an FGF receptor) was compulsively expressed by means of genetic engineering. This *Digenea simplex* extract exhibited a similar cell growth promoting activity to FGF18 on FGFR4-expressing cells, but exhibited no cell growth promoting activity on the parent cell strain which does not express FGFR4. Thus, the *Digenea simplex* extract can be said to be a FGF18-like active substance.

Further, as expected, this FGF18-like active substance was demonstrated to have a hair regrowth inhibiting effect (see Example 6).

With respect to the cell growth promoting effect on FGFR4-expressing cells, an experiment was performed to compare the effect of using FGF 18 alone with that of using FGF18 together with *Digenea simplex* extract. The results confirmed that low concentrations of *Digenea simplex* extract promote the activity of FGF18. Therefore, *Digenea simplex* extract can be said to be a substance that promotes the activity of FGF 18. From these results, it can be seen that the technique of this comparative experiment may be used as a screening method for substances that promote the activity of FGF 18. It is evident that this technique is a promising means for selecting candidates for hair regrowth inhibiting agents.

EXAMPLES

Hereinbelow, the present invention will be described with reference to the following Examples. However, the technical scope of the present invention is not limited to these Examples.

[EXAMPLE 1]

Effect of FGF18 on Gene Expression in Dermal Papilla Cells

In this Example, in order to evaluate the function of FGF18 on hair growth, the effect of FGF18 on gene expression was examined in dermal papilla cells which are believed to be a control tower for hair follicle growth.

<Materials and Methods>

Cultured human dermal papilla cells (HFDP; from adult human scalp; Toyobo) were subcultured in HFDP growth medium (20% fetal bovine serum-containing basal medium for dermal papilla cell; Toyobo) and used in experiments within two passages. The cells were treated with trypsin and seeded in 24-well collagen-coated plates (Sumilon) at a density of $1x10^4$ cells/well. The cells were maintained at 37°C in HFDP medium. On the next day, FGF18 was added to the medium. After the cells were cultured therein for 24 hr, the medium was exchanged for FGF18 free medium. Control group was cultured in FGF18 free medium. Subsequently, the cells were harvested and the mRNA was extracted and purified. The expression level of mRNA from VEGF (known as a hair follicle growth promoting factor) contained in the resultant mRNA was analyzed.

<Results>

The results are shown in Fig. 1. It is believed that dermal papilla cells release various factors to thereby support hair growth. On of such factors is VEGF. In this experiment, addition of FGF18 to the medium decreased the expression level of *VEGF* mRNA in dermal papilla cells. Therefore, it was confirmed that FGF18 inhibits the production of one factor for hair growth that is released by dermal papilla cells.

[EXAMPLE 2]

Inhibitory Effect of FGF18 on Hair Growth

In this Example, in order to examine the effect of FGF18 *in vivo* on hair follicle growth, the effect of administering GF18 was tested on C3H/HeN mice that had been induced to a hair follicle anagen phase.

<Materials and Methods>

In order to examine the effect of FGF18 *in vivo* on hair follicle growth, FGF18 protein dissolved in phosphate buffered physiological saline (PBS) was administered to hair follicle anagen phase-induced mice.

Briefly, dorsal hair of 50 day-old C3H/HeN male mice in telogen phase was depilated gently with fingers to thereby induce the start of hair follicle anagen phase. Then, FGF18 solution was injected into the dorsal skin subcutaneously from the vicinity of the tail (1 μg of FGF18 per mouse). The mice were maintained on a diet and water *ad libitum.* Starting from this day, FGF18 solution was injected subcutaneously into the dorsal skin every day at about the same time for 8 days. The control group received injection of PBS instead of FGF18. Nine days after the initial injection, the mice were euthanized under anesthesia. Full thickness skin samples were excised from the dorsal part and embedded in paraffin. The thus embedded skin samples were sliced into 4 μm thick sections with a microtome, stained with hematoxylin and observed under microscope.

<Results>

The results are shown in Fig. 2. In this Figure, A represents a microphotograph of a skin section from a control mouse which received PBS; B represents a microphotograph of a skin section from an FGF 18-administered mouse. In A and B, the magnification is the same.

As seen from microphotograph A, natural growth of hair follicles was observed at day 9 in the PBS-administered mouse. Hair follicles had grown long and reached the lower layer of the skin.

On the other hand, in the FGF18 solution-administered mouse in microphotograph B, hair follicles are short, suggesting that hair growth is strongly inhibited.

From these results, it was demonstrated *in vivo* that continuous administration of FGF18 is capable of inhibiting the growth of hair follicles.

[Example 3]

FGF Receptor Stimulating Effect of FGF18 Partial Peptides

As FGF18 partial peptides, partial polypeptides were prepared based on SEQ ID NO: 14 which corresponds to the amino acid sequence of mouse FGF18. Briefly, amino acids from position 4 to position 95 as counted from N-terminus (excluding methionine) were deleted to prepare partial peptides d4-d95 [indicating the number of amino acids deleted from N-terminal (excluding methionine)]. As additional FGF18 partial peptides, partial polypeptides were prepared based on SEQ ID NO: 14 which corresponds to the amino acid sequence of mouse FGF 18 by deleting amino acids from position 8 to position 25 as counted from C-terminus. Thus obtained were partial peptides dc8-dc25 [indicating the number of amino acids deleted from C-terminal].

The amino acid sequences (nucleotide sequences) of the individual partial peptides correspond to the following SEQ ID NOS.

d4: SEQ ID NO: 15 (SEQ ID NO: 4)
d12: SEQ ID NO: 16 (SEQ ID NO: 5)
d16: SEQ ID NO: 17 (SEQ ID NO: 6)
d18: SEQ ID NO: 18 (SEQ ID NO: 7)
d22: SEQ ID NO: 19 (SEQ ID NO: 8)

d37: SEQ ID NO: 20 (SEQ ID NO: 9)
d48: SEQ ID NO: 21 (SEQ ID NO: 10)
d67: SEQ ID NO: 22 (SEQ ID NO: 11)
d77: SEQ ID NO: 23 (SEQ ID NO: 12)
d95: SEQ ID NO: 24 (SEQ ID NO: 13)
dc8: SEQ ID NO: 28 (SEQ ID NO: 25)
dc15: SEQ ID NO: 29 (SEQ ID NO: 26)
dc25: SEQ ID NO: 30 (SEQ ID NO: 27)

FGF18 stimulates the four FGF receptors FGFR1c, FGFR2c, FGFR3c and FGFR4, but the intensity of stimulation varies depending on the receptor. It is believed that summation of stimulations on these receptors results in inhibition of hair growth.

According to teachings disclosed in literature, the FGF receptor genes *FGFR1c, FGFR2c, FGFR3c* and *FGFR4* were respectively introduced by means of genetic engineering into Ba/F3 cell strain (obtained from RIKEN BRC) which is mouse IL-3-dependent proB cell to thereby prepare cells on whose surface FGFR was compulsively expressed (Omitz, DM., Xu, J., Colvin, JS., McEwen, DG, MacArthur, CA., Coulier, F., Gao, G and Goldfarb, M., 1996. Receptor specificity of the fibroblast growth factor family. J. Biol. Chem. 271(25):15292-15297; Yoneda, A., Asada, M., Oda, Y, Suzuki, M. and Imamura, T., 2000. Engineering of an FGF-proteoglycan fusion protein with heparin-independent, mitogenic activity. Nat. Biotec. 18(6):641-644).

Using these cells, the individual receptor stimulating activities were examined. The results are shown in Fig. 3.

Test results using FGFR1c expressing cell (R1c), FGFR2c expressing cell (R2c), FGFR3c expressing cell (R3c) and FGFR4 expressing cell (R4) are shown in this order in Fig. 3, with R1c on the left side.

The longitudinal axis represents the DNA synthesis level when cells were stimulated with about 100 ng/ml of a sample, taking the basal DNA synthesis level for no cell stimulation as 100%.

<R1c>
Column 1: no sample (control). Column 2: (d4). Column 3: (d12). Column 4: (d18). Column 5: (d22). Column 6: (d37). Column 7: (d77). Column 8: (d95)
<R2c>
Column 9: no sample (control). Column 10: (d4). Column 11: (d22). Column 12: (d48). Column 13: (d67). Column 14: (d77). Column 15: (d95).
<R3c>
Column 16: no sample (control). Column 17: (d4). Column 18: (d12). Column 19: (d16). Column 20: (d18). Column 21: (d22). Column 22: (d37). Column 23: (d67). Column 24: (d77). Column 25: (d95).
<R4>
Column 26: no sample (control). Column 27: (d4). Column 28: (d12). Column 29: (d16). Column 30: (d18). Column 31: (d22). Column 32: (d37). Column 33: (d67). Column 34: (d77).

Fig. 4 shows the test results using FGFR2c expressing cell (R2c), FGFR3c expressing cell (R3c) and FGFR4 expressing cell (R4) in this order, with R2c on the left side.

The longitudinal axis represents the DNA synthesis level in CPM when cells were stimulated with various concentrations of samples, taking the basal DNA synthesis level for no cell stimulation as 0 CPM. The horizontal axis represents the concentration of each sample used to stimulate cells.

<R2c>
Upper graph: full length (control). Center graph: (dc8). Lower graph: (dc25)
<R3c>
Upper graph: full length (control). Center graph: (dc8). Lower graph: (dc25)
<R4>
Upper graph: full length (control) Center graph: (dc8). Lower graph: (dc25)

From Fig. 3 and Fig. 4, even FGF18 partial peptides (d4-d95, dc8-dc25) are recognized to have an activity of stimulating FGF receptors with various intensities and thereby stimulating the DNA synthesis by cells. In particular, FGF18 partial peptides were shown to have an activity of strongly stimulating FGFR3c expressing cell and FGFR4 expressing cell. These experimental results, showing that FGF18 partial peptides have an activity similar to the FGF receptor stimulating activity of FGF18, indicate that these partial peptides also have an FGF18-like activity in hair growth regulation.

Among the FGF18 partial peptides, d4-d37 and dc8-dc25, especially d4-d22 and dc8-dc25 exhibited strong receptor stimulating activity on both FGFR3c and FGFR4. Therefore, the partial peptides where up to 37 amino acids, especially up to 22 amino acids (excluding methionine) are deleted from the N-terminal side, and the partial peptide where up to 25 amino acids are deleted from the C-terminal side are preferably used as the FGF18 of the present invention.

[EXAMPLE 4]

Screening for FGF18-Like Active Substances Using FGFR4 Expressing Cell

The cell on whose surface FGR4 is compulsively expressed (R4/Ba/F3 cell) prepared above was cultured in the presence of various plant extracts as test solutions. As positive control, a commercial FGF 18 protein was used. The cell count after culturing for a specific time period was determined with Cell Counting Kit-8 (manufactured by Dojindo Laboratories and sold by Wako Pure Chemical Industries) by measuring the coloring at 450 nm which was proportionate to the yield of WST-8 formazan.

As a result, it was found that *Digenea simplex* extract acts as FGF18-like active substance and is capable of promoting the growth of R4Ba/F3 cell.

[EXAMPLE 5]

Cell Growth Promoting Activity of the FGF18-Like Active Substance of the Invention

To 2.0 g of dried *Digenea simplex* (Ryukyu Marine Product Processing Plant, or Nago Nori Shokai), 40 ml of 70% ethanol aqueous solution was added. Then, soaking extraction was performed at room temperature for 7 days. To 2.0 g of dried *Digenea simplex* (Ryukyu Marine Product Processing Plant, or Nago Nori Shokai), 40 ml of distilled water was added. The resultant mixture was boiled for 20 min. Crude extracts obtained from these procedures were filtered, and the filtrates were collected to give extracts.

In the same manner as in Example 3, the cell growth promoting activity of the FGF18-like active substance of the present invention was measured using R4Ba/F3 cell. Specifically, measurement was performed as described below. Briefly, RPMI1640 medium containing 10% FBS and 1% Antibiotic G-418 Sulfate (Promega; V7983) was added to each well of 96-well cell culture plates (50 μl/well). Subsequently, various concentrations of test solutions prepared by diluting samples with water were added (10 μl/well), followed by addition of 50 μl of cell suspension in which $5x10^4$ R4Ba/F3 cells were suspended in RPMI1640 medium containing 10% FBS and 1% Antibiotic G-418 Sulfate. The resultant mixture was stirred lightly. Finally, 10 μl of heparin/10% FBS/1% Antibiotic G-418 Sulfate/RPMI1640 medium (final heparin concentration: 5 μg/ml) was added. Then, the cell was cultured in a carbon dioxide incubator at 37°C under 5% $CO_2$ for 72 hr. To determine the growth of R4Ba/F3 cell, 10 μl of Cell Counting Kit-8 (manufactured by Dojindo Laboratories and sold by Wako Pure Chemical Industries)/PBS solution was added to each well after 72-hr culture, followed by culturing for another 3 hrs, and the coloring at 450 nm which was proportionate to the yield of WST-8 formazan was measured.

In the measurement, 10 μl of FGF18 (PeproTech; 100-28) solution (final FGF18 concentration: 3 ng/ml) was used as a positive control. As a negative control, 10 μl of water or ethanol solution (final concentration of ethanol was adjusted to 1% or less) used for preparing test solutions was used. Cell proliferation rate (%) was calculated by measuring the absorbance obtained when the test solution was added. The absorbance obtained when FGF18 (final concentration: 3 ng/ml) as the positive control had been added was taken as 100%; and the absorbance obtained when water or ethanol solution as the negative control had been added was taken as 0%.

The results on *Digenea simplex* extracts are shown in Fig. 5. When *Digenea simplex* extract of final concentration 8.3% was used as a test solution, the amount of coloring increased. Thus, it was confirmed that this *Digenea simplex* extract has an FGF18-like cell growth promoting activity.

In Fig. 5, *Digenea simplex* extract exhibited a stronger cell growth promoting effect at concentrations 0.083% and 0.83%, compared to the case where FGFR4BaF3 strain was cultured in the presence of FGF18 alone. Thus, *Digenea simplex* extract of low concentrations has an FGF18 activity promoting effect.

In short, *Digenea simplex* extract is both an FGF-like active substance and a substance that promotes FGF18 activity.

[EXAMPLE 6]

In order to examine the *in vivo* effect of the FGF18-like active substance, a test was performed on C3H/HeN mice in telogen phase of the hair cycle.

*In vivo* Analysis of the FGF 18-like Active Substance of the Invention

In the same manner as described in Example 5, 30 ml of distilled water was added to 2.0 g of dried *Digenea simplex* (Ryukyu Marine Product Processing Plant, or Nago Nori Shokai). The resultant mixture was boiled for 20 min. After the extract returned to room temperature, it was centrifuged at 12,000 rpm for 10 min to give a supernatant. This supernatant was filtered through a 0.45 mm Millex HV sterilization filter to make a test solution. The thus obtained *Digenea simplex* extract was administered subcutaneously to the dorsal skin of C3H/HeN mice in telogen phase of the hair cycle. After anesthetizing, dorsal hair of 7 week-old C3H/HeN male mice was depilated gently with a hair clipper. After depilation, 100 μl of the *Digenea simplex* extract as test solution or PBS(-) (physiological phosphate buffer) was injected subcutaneously into 5 mice per group (day 0). In a similar manner, subcutaneous injection was performed again at day 4. The state of hair regrowth in the depilated area in the back of mice was observed with eyes at day 21, day 28 and day 35, to thereby give hair regrowth scores. Each state was scored as follows: 1) pigmentation: 1 point; 2) short hair: 2 points; 3) usual hair: 3 points. The ratio of the area of each hair rebirth state to the total depilated area was determined in %. Then, hair regrowth score was calculated by the formula described below. According to this calculation method, the score is 100 when the total depilated area has been recovered to usual hair state.

$$\text{Hair regrowth score} = [\text{ratio of pigmentation area (\%)} \times 1 + \text{ratio of short hair area (\%)} \times 2 + \text{ratio of usual hair area (\%)} \times 3]/3$$

The state of hair regrowth in the depilated area in the back of mice was observed at day 21 (3w), day 28 (4w) and day 35 (5w) after the first subcutaneous injection. As a result, as shown in Fig. 6, *Digenea simplex* extract administered group exhibited lower hair regrowth scores than PBS(-) administered group. Thus, it was demonstrated *in vivo* that *Digenea simplex* extract is capable of inhibiting hair growth.

As described so far, the effect of *Digenea simplex* as a hair regrowth inhibiting agent has been demonstrated.

[EXAMPLE 7]

A formulation of a hair shampoo of the present invention comprising a *Digenea simplex* extract and a method of preparing the shampoo are described below.

A hair shampoo was prepared according to the following formulation and preparation method.

(Formulation)

| Component | Weight % |
|---|---|
| 1. Diluted solution obtained in Example 5 | 0.1 |
| 2. Sodium laurylether sulfate ethanol | 20 |
| 3. Sodium lauryl sulfate | 10 |
| 4. 1,3-Butylene glycol | 1 |
| 5. Flavor | proper quantity |
| 6. Purified water | to make the total 100 |

(Preparation Method)

The components listed above were heated to 80°C, mixed by stirring and then cooled under stirring. Thus, the shampoo of the present invention was prepared.

[EXAMPLE 8]

A formulation of a hair liquid of the present invention comprising a *Digenea simplex* extract and a method of preparing the hair liquid are described below.

A hair liquid was prepared according to the following formulation and preparation method.

(Formulation)

| Component | Weight % |
|---|---|
| 1. Diluted solution obtained in Example 5 | 0.1 |
| 2. Ethanol | 40 |
| 3. Glycerol | 1 |
| 4. Flavor | proper quantity |
| 5. Purified water | to make the total 100 |

(Preparation Method)

The components listed above other than purified water were dissolved by stirring and then purified water was added. Thus, the hair liquid of the present invention was prepared.

[EXAMPLE 9]

A formulation of a hair cream of the present invention comprising a *Digenea simplex* extract and a method of preparing the hair cream are described below.

A hair cream was prepared according to the following formulation and preparation method.

(Formulation)

| Component | Weight % |
|---|---|
| 1. Diluted solution obtained in Example 5 | 0.1 |
| 2. Liquid paraffin | 40 |
| 3. Vaseline | 1 |
| 4. Cetostearyl alcohol | 1 |
| 5. Methyl polysiloxane | 1 |
| 6. Methyl paraoxybenzoate | 0.2 |
| 7. Propylene glycol | 5 |
| 8. Flavor | proper quantity |
| 9. Purified water | to make the total 100 |

(Preparation Method)

The components listed above were mixed by stirring to thereby prepare the hair cream of the present invention.

INDUSTRIAL APPLICABILITY

According to the present invention, effective hair growth inhibiting agents and hair removing agents are provided. By incorporating these agents, it is possible to provide shampoos and hair liquids with hair growth inhibiting activity, as well as depilating creams for removing unwanted hair from the skin.

## Claims

1. A method of non-therapeutic hair growth inhibition or non-therapeutic hair removal comprising administering FGF18 and/or *Digenea simplex* extract and/or an expression vector carrying an FGF18-encoding DNA to hair follicles which are in the anagen phase.

2. The method according to claim 1 wherein said FGF18 and/or *Digenea simplex* extract and/or expression vector carrying an FGF18-encoding DNA is used in combination with another hair growth-inhibiting agent or hair removing agent.

3. The method according to claim 1 or 2 wherein said FGF18 and/or extract and/or expression vector carrying an FGF18-encoding DNA is administered so that the FGF18 and/or *Digenea simplex* extract and/or expression vector carrying an FGF18-encoding DNA remains in hair follicles at a concentration of at least 1% of the initial concentration for a period of at least 1 day.

4. The method according to any one of the preceding claims wherein said anagen phase has been induced by depilating.

5. A method of non-therapeutic hair growth inhibition or non-therapeutic hair removal comprising the steps of:

(i) inducing anagen phase in hair follicles; and
(ii) administering to said hair follicles FGF18 and/or *Digenea simplex* extract and/or an expression vector carrying an FGF18-encoding DNA.

**6.** The method according to any one of the preceding claims, wherein:

(a) the expression vector carrying an FGF18-encoding DNA integrated thereinto is being administered so that the FGF18 is expressed in hair follicles continuously; or
(b) said FGF18 is a full-length peptide of the amino acid sequence as shown in SEQ ID NO: 2 or a partial peptide there of having FGF18-like activity.

**7.** FGF18 and/or *Digenea simplex* extract and/or an expression vector carrying an FGF18-encoding DNA for use in a method of therapeutic hair growth inhibition or therapeutic hair removal wherein said FGF18 and/or extract and/or expression vector carrying an FGF18-encoding DNA is administered to hair follicles which are in anagen phase.

**8.** The FGF18 and/or extract and/or expression vector carrying an FGF18-encoding DNA for use according to claim 7 wherein said FGF18 and/or *Digenea simplex* extract and/or expression vector carrying an FGF18-encoding DNA is used in combination with another hair growth-inhibiting agent or hair removing agent.

**9.** The FGF18 and/or extract and/or expression vector carrying an FGF18-encoding DNA for use according to claim 7 or 8 wherein said FGF18 and/or *Digenea simplex* extract and/or expression vector carrying an FGF18-encoding DNA is administered so that the FGF18 and/or *Digenea simplex* extract and/or expression vector carrying an FGF18-encoding DNA remains in hair follicles at a concentration of at least 1% of the initial concentration for a period of at least 1 day.

**10.** The FGF18 and/or extract and/or expression vector carrying an FGF18-encoding DNA for use according to any one of claims 7 to 9 wherein said anagen phase has been induced by depilating.

**11.** The FGF 18 and/or extract and/or expression vector carrying an FGF 18-encoding DNA for use according to any one of claims 7 to 10 wherein:

(a) the expression vector carrying an FGF18-encoding DNA integrated thereinto is being administered so that the FGF18 is expressed in hair follicles continuously; or
(b) said FGF18 is a full-length peptide of the amino acid sequence as shown in SEQ ID NO: 2 or a partial peptide there of having FGF18-like activity.

**12.** A method of screening for a substance that promotes the hair growth inhibiting activity of FGF18 as defined in any one of claims 1 to 6 to thereby obtain candidates for the hair growth-inhibiting agent or hair removing agent from test substances, said method comprising the following steps (a) to (d):

(a) preparing a non-human experimental animal or a cultured animal cell capable of expressing *FGF18* to an observable extent,
(b) bringing a test substance into contact with the experimental animal, or the cultured animal cell,
(c) monitoring the expression of *FGF18* in the experimental animal or the cultured animal cell after step (b), and
(d) selecting those test substances which have a function of promoting the expression of *FGF18*.

**13.** The method according to claim 12, wherein the expression of *FGF18* is monitored in step (c) by extracting mRNA from the experimental animal or the cultured animal cell after step (b) and then analyzing the mRNA level of expressed *FGF18;* and those test substances which have a function of promoting the expression of *FGF18* are selected in step (d) by selecting systems that exhibited higher levels of *FGF18* mRNA than when the test substance was not allowed to act.

**Patentansprüche**

**1.** Verfahren zur nichttherapeutischen Haarwachstumshemmung oder nichttherapeutischen Haarentfernung, das Verabreichen von FGF18 und/oder *Digenea simplex*-Extrakt und/oder einem Expressionsvektor, der eine FGF18-codierende DNA trägt, an Haarfollikel, die in der Anagenphase sind.

**2.** Verfahren gemäß Anspruch 1, wobei das FGF18 und/oder der *Digenea simplex*-Extrakt und/oder der Expressionsvektor, der eine FGF18-codierende DNA trägt, in Kombination mit einem anderen Haarwachstum hemmenden Mittel oder Haar entfernenden Mittel verwendet wird/werden.

**3.** Verfahren gemäß Anspruch 1 oder 2, wobei das FGF18 und/oder der Extrakt und/oder der Expressionsvektor, der eine FGF18-codierende DNA trägt, so verabreicht wird/werden, dass das FGF18 und/oder der *Digenea simplex*-Extrakt und/oder der Expressionsvektor, der eine FGF18-codierende DNA trägt, für einen Zeitraum von wenigstens einem Tag in einer Konzentration von wenigstens 1% der Anfangskonzentration in Haarfollikeln bleibt/bleiben.

**4.** Verfahren gemäß einem der vorangehenden Ansprüche, wobei die Anagenphase durch Depilieren induziert wurde.

**5.** Verfahren zur nichttherapeutischen Haarwachstumshemmung oder nichttherapeutischen Haarentfernung, umfassend die Schritte:

(i) Induzieren der Anagenphase in Haarfollikeln und
(ii) Verabreichen von FGF18 und/oder *Digenea simplex*-Extrakt und/oder einem Expressionsvektor, der eine FGF18-codierende DNA trägt, an die Haarfollikel.

**6.** Verfahren gemäß einem der vorangehenden Ansprüche, wobei:

(a) der Expressionsvektor, der eine FGF18-codierende DNA integriert hat, so verabreicht wird, dass das FGF18 kontinuierlich in Haarfollikeln exprimiert wird, oder
(b) das FGF18 ein Volllängenpeptid der Aminosäuresequenz, wie sie in SEQ ID NO:2 gezeigt ist, oder ein Partialpeptid davon mit FGF18-artiger Aktivität ist.

**7.** FGF18 und/oder *Digenea simplex*-Extrakt und/oder Expressionsvektor, der eine FGF18-codierende DNA trägt, zur Verwendung in einem Verfahren zur therapeutischen Haarwachstumshemmung oder therapeutischen Haarentfernung, wobei das FGF18 und/oder der Extrakt und/oder der Expressionsvektor, der eine FGF18-codierende DNA trägt, an Haarfollikel, die in der Anagenphase sind, verabreicht wird/werden.

**8.** FGF18 und/oder Extrakt und/oder Expressionsvektor, der eine FGF18-codierende DNA trägt, zur Verwendung gemäß Anspruch 7, wobei das FGF18 und/oder der *Digenea simplex*-Extrakt und/oder der Expressionsvektor, der eine FGF18-codierende DNA trägt, in Kombination mit einem anderen Haarwachstum hemmenden Mittel oder Haar entfernenden Mittel verwendet wird/werden.

**9.** FGF18 und/oder Extrakt und/oder Expressionsvektor, der eine FGF18-codierende DNA trägt, zur Verwendung gemäß Anspruch 7 oder 8, wobei das FGF18 und/oder der Expressionsvektor, der eine FGF18-codierende DNA trägt, so verabreicht werden/wird, dass das FGF18 und/oder der *Digenea simplex*-Extrakt und/oder der Expressionsvektor, der eine FGF18-codierende DNA trägt, in einer Konzentration von wenigstens 1% der Anfangskonzentration für einen Zeitraum von wenigstens einem Tag in Haarfollikeln bleiben/bleibt.

**10.** FGF18 und/oder Extrakt und/oder Expressionsvektor, der eine FGF18-codierende DNA trägt, zur Verwendung gemäß einem der Ansprüche 7 bis 9, wobei die Anagenphase durch Depilieren induziert worden ist.

**11.** FGF18 und/oder Extrakt und/oder Expressionsvektor, der eine FGF18-codierende DNA trägt, zur Verwendung gemäß einem der Ansprüche 7 bis 10, wobei:

(a) der Expressionsvektor, der eine FGF18-codierende DNA darin integriert trägt, so verabreicht wird, dass das FGF18 kontinuierlich in Haarfollikeln exprimiert wird, oder
(b) das FGF18 ein Volllängenpeptid der Aminosäuresequenz, wie in SEQ ID NO:2 gezeigt, oder ein Partialpeptid davon mit FGF18-artiger Aktivität ist.

**12.** Verfahren zum Screening auf eine Substanz, die die Haarwachstum hemmende Aktivität von FGF18, wie in einem der Ansprüche 1 bis 6 definiert, begünstigt, um dadurch Kandidaten für das Haarwachstum hemmende Mittel oder Haar entfernende Mittel aus Testsubstanzen zu erhalten, wobei das Verfahren die folgenden Schritte (a) bis (d) umfasst:

(a) Präparieren eines nicht-menschlichen Versuchstiers oder einer kultivierten Tierzelle, das/die fähig ist, FGF18 zu einem beobachtbaren Grad zu exprimieren,
(b) Inkontaktbringen einer Testsubstanz mit dem Versuchstier oder der kultivierten Tierzelle,
(c) Überwachen der Expression von FGF18 in dem Versuchstier oder der kultivierten Tierzelle nach Schritt (b) und
(d) Selektieren solcher Testsubstanzen, die eine Funktion zur Begünstigung der Expression von FGF18 haben.

**13.** Verfahren gemäß Anspruch 12, wobei die Expression von FGF18 in Schritt (c) überwacht wird, indem mRNA aus dem Versuchstier oder der kultivierten Tierzelle nach Schritt (b) extrahiert wird und dann der mRNA-Level von exprimiertem FGF18 analysiert wird, und solche Testsubstanzen, die eine Funktion zur Begünstigung der Expression von FGF18 haben, in Schritt (d) durch Selektiersysteme selektiert werden, die höhere Level an FGF18-mRNA aufwiesen als in dem Fall, in dem die Testsubstanz nicht wirken gelassen wurde.

**Revendications**

**1.** Procédé d'inhibition non-thérapeutique de la pousse des poils ou d'élimination non-thérapeutique des poils, comportant le fait d'administrer, à des follicules pileux en phase anagène, du facteur de croissance FGF18 et/ou un extrait de *Digenea simplex* et/ou un vecteur d'expression qui comporte un ADN codant le FGF18.

**2.** Procédé conforme à la revendication 1, dans lequel on utilise le FGF18 et/ou l'extrait de *Digenea simplex* et/ou le vecteur d'expression comportant un ADN codant le FGF18 en combinaison avec un autre agent d'inhibition de la pousse des poils ou d'élimination des poils.

**3.** Procédé conforme à la revendication 1 ou 2, dans lequel on administre le FGF18 et/ou l'extrait de *Digenea simplex* et/ou le vecteur d'expression qui comporte un ADN codant le FGF18 de telle manière que le FGF18 et/ou l'extrait de *Digenea simplex* et/ou le vecteur d'expression qui comporte un ADN codant le FGF18 reste(nt), durant un laps de temps d'au moins 1 jour, présent(s) dans les follicules pileux en une concentration représentant au moins 1 % de la concentration initiale.

**4.** Procédé conforme à l'une des revendications précédentes, pour lequel ladite phase anagène a été induite par dépilation.

**5.** Procédé d'inhibition non-thérapeutique de la pousse des poils ou d'élimination non-thérapeutique des poils, comportant les étapes suivantes :

i) induire la phase anagène de follicules pileux,
ii) et administrer à ces follicules pileux du FGF18 et/ou un extrait de *Digenea simplex* et/ou un vecteur d'expression qui comporte un ADN codant le FGF18.

**6.** Procédé conforme à l'une des revendications précédentes, dans lequel

a) le vecteur d'expression qui comporte, intégré en son sein, un ADN codant le FGF18 est administré de manière à ce que le FGF18 soit exprimé continuellement dans les follicules pileux ;
b) ou ledit FGF18 est un peptide qui comporte la séquence entière d'acides aminés de la Séquence N° 2 ou un peptide qui en est une partie dotée d'une activité de type FGF18.

**7.** Facteur de croissance FGF18 et/ou extrait de *Digenea simplex* et/ou vecteur d'expression comportant un ADN codant le FGF18, pour utilisation dans un procédé d'inhibition thérapeutique de la pousse des poils ou d'élimination thérapeutique des poils, dans lequel ledit FGF18 et/ou extrait de *Digenea simplex* et/ou vecteur d'expression comportant un ADN codant le FGF18 est administré à des follicules pileux qui sont en phase anagène.

**8.** Facteur de croissance FGF18 et/ou extrait et/ou vecteur d'expression comportant un ADN codant le FGF18 pour utilisation conforme à la revendication 7, dans laquelle ledit FGF18 et/ou ledit extrait de *Digenea simplex* et/ou ledit vecteur d'expression comportant un ADN codant le FGF18 est ou sont utilisé(s) en combinaison avec un autre agent d'inhibition de la pousse des poils ou d'élimination des poils.

**9.** Facteur de croissance FGF18 et/ou extrait et/ou vecteur d'expression comportant un ADN codant le FGF18 pour utilisation conforme à la revendication 7 ou 8, dans laquelle ledit FGF18 et/ou ledit vecteur d'expression comportant un ADN codant le FGF18 est ou sont administré(s) de telle manière que le FGF18 et/ou l'extrait de *Digenea simplex* et/ou le vecteur d'expression comportant un ADN codant le FGF18 reste(nt), durant un laps de temps d'au moins 1 jour, présent(s) dans les follicules pileux en une concentration représentant au moins 1 % de la concentration initiale.

**10.** Facteur de croissance FGF18 et/ou extrait et/ou vecteur d'expression comportant un ADN codant le FGF18 pour utilisation conforme à l'une des revendications 7 à 9, pour laquelle ladite phase anagène a été induite par dépilation.

**11.** Facteur de croissance FGF18 et/ou extrait et/ou vecteur d'expression comportant un ADN codant le FGF18 pour utilisation conforme à l'une des revendications 7 à 10, dans laquelle

a) le vecteur d'expression qui comporte, intégré en son sein, un ADN codant le FGF18 est administré de manière à ce que le FGF18 soit exprimé continuellement dans les follicules pileux ;
b) ou ledit FGF18 est un peptide qui comporte la séquence entière d'acides aminés de la Séquence N° 2 ou un peptide qui en est une partie dotée d'une activité de type FGF18.

**12.** Procédé de recherche par criblage d'une substance qui promeuve l'activité d'inhibition de la pousse de poils du facteur FGF18, telle que définie dans l'une des revendications 1 à 6, dans le but d'obtenir ainsi, parmi les substances testées, des candidats au titre d'agent d'inhibition de la pousse des poils ou d'agent d'élimination des poils, lequel procédé comporte les étapes (a) à (d) suivantes :

a) préparer un animal d'expérience non-humain ou une cellule animale en culture, capable d'exprimer le facteur FGF18 à un niveau détectable,
b) mettre une substance à tester en contact avec l'animal d'expérience ou avec la cellule animale en culture,
c) surveiller l'expression du facteur FGF18 chez l'animal d'expérience ou chez la cellule animale en culture, après l'étape (b),
d) et sélectionner les substances testées qui possèdent une fonction de promotion de l'expression du facteur FGF18.

**13.** Procédé conforme à la revendication 12, dans lequel, dans l'étape (c), on surveille l'expression du facteur FGF18 en extrayant de l'ARNm, après l'étape (b), de l'animal d'expérience ou de la culture de cellule animale, et en analysant ensuite le niveau d'ARNm de FGF18 exprimé, et dans lequel on sélectionne, dans l'étape (d), les substances testées qui possèdent une fonction de promotion de l'expression du facteur FGF18 en sélectionnant les systèmes qui donnent des niveaux d'ARNm de FGF18 plus élevés que lorsque l'on ne fait pas agir la substance testée.

Fig. 1

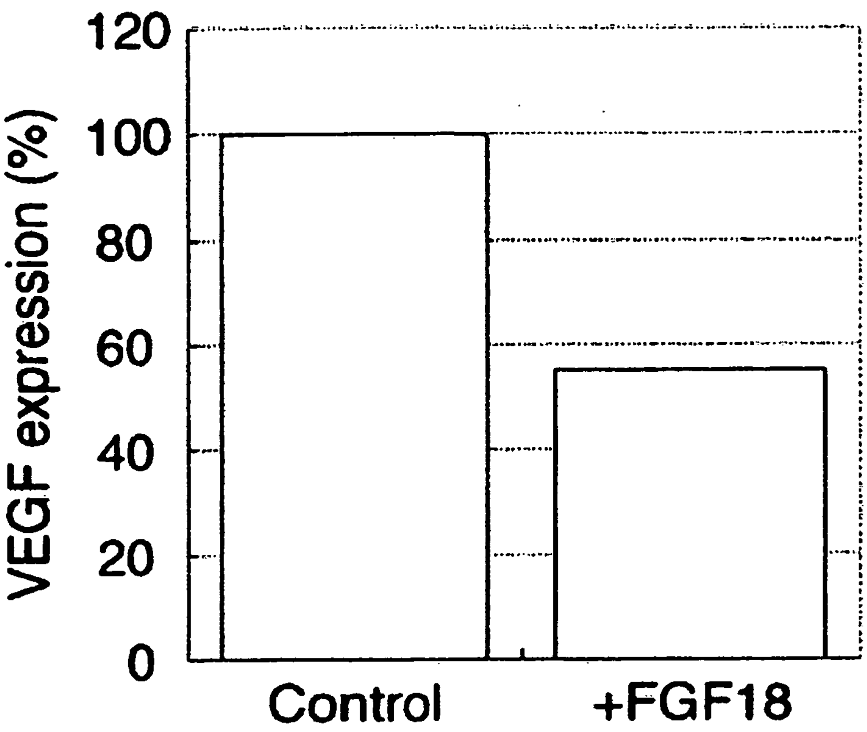


Fig. 2

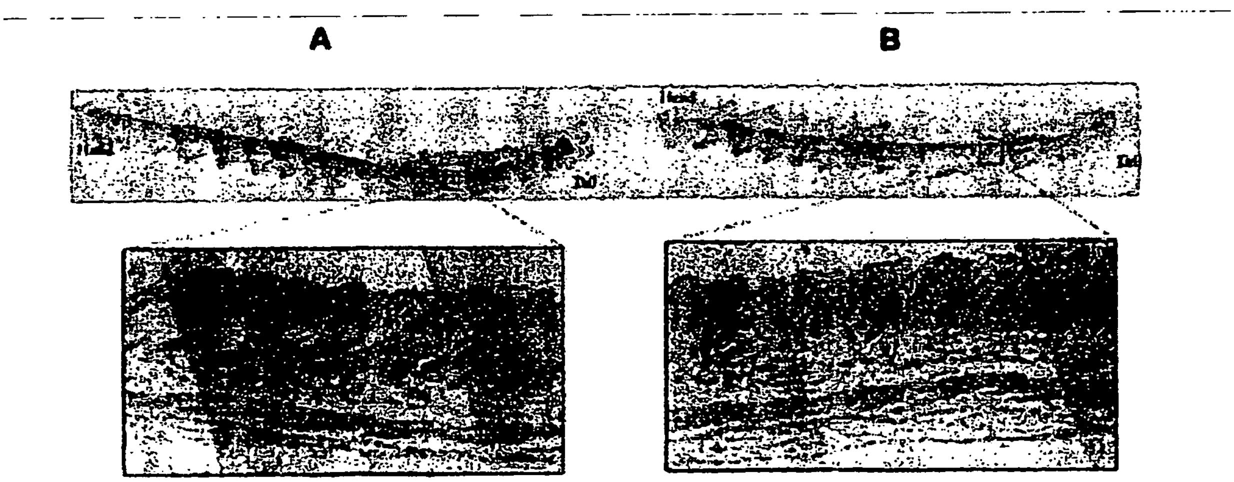

Fig. 3

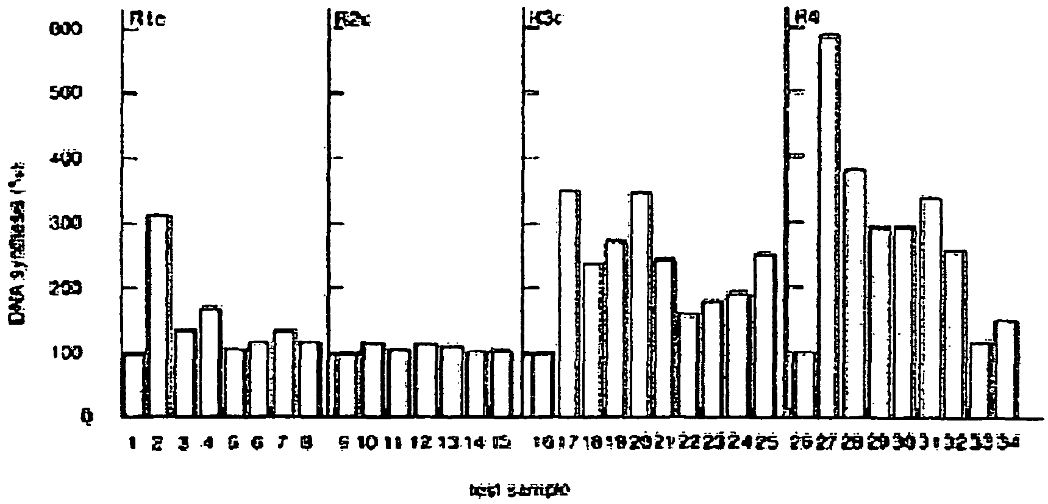
R1c
R2c
R3c
R4
600
500
400
300
200
100
0
DNA synthesis (%)
1 2 3 4 5 6 7 8 9 10 11 12 13 14 15 16 17 18 19 20 21 22 23 24 25 26 27 28 29 30 31 32 33 34
test sample

Fig. 4

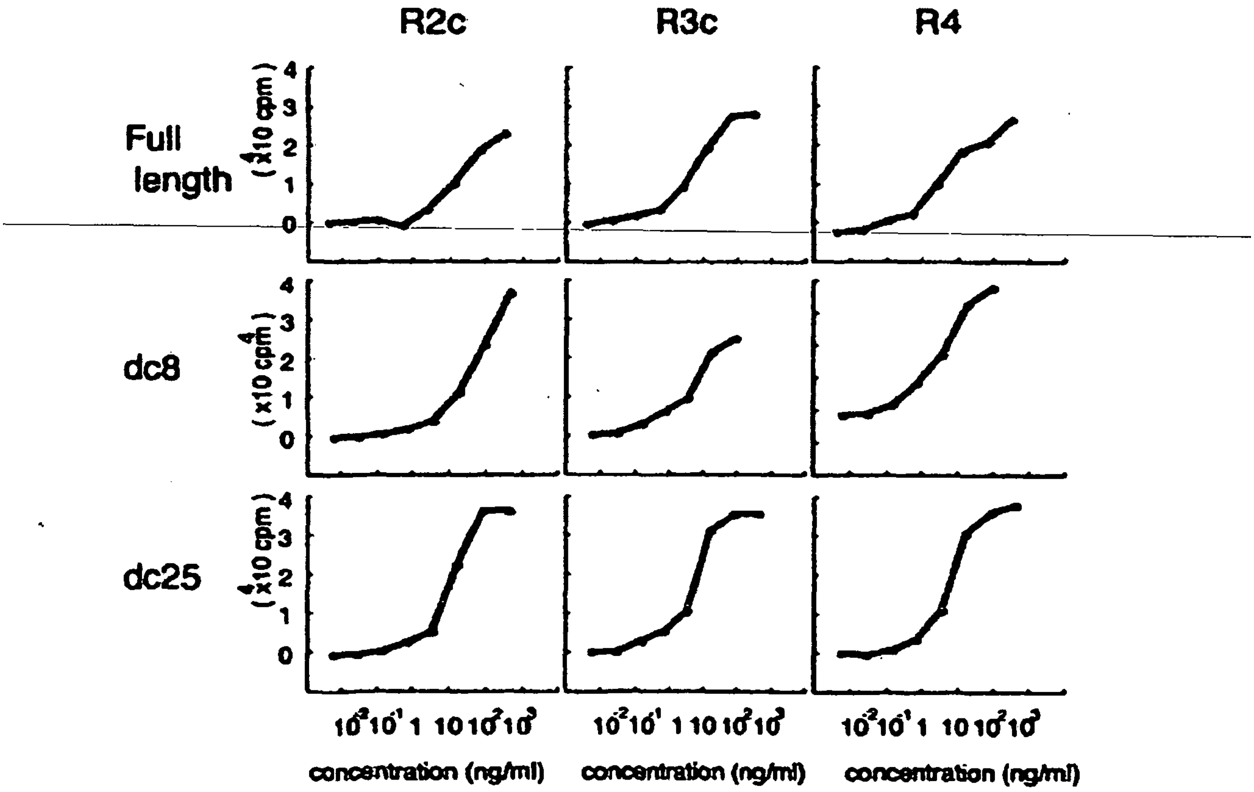
R2c
R3c
R4
Full length
dc8
dc25
($\times 10^4$ cpm)
0
1
2
3
4
$10^{-2}$ $10^{-1}$ 1 10 $10^2$ $10^3$
concentration (ng/ml)

Fig. 5

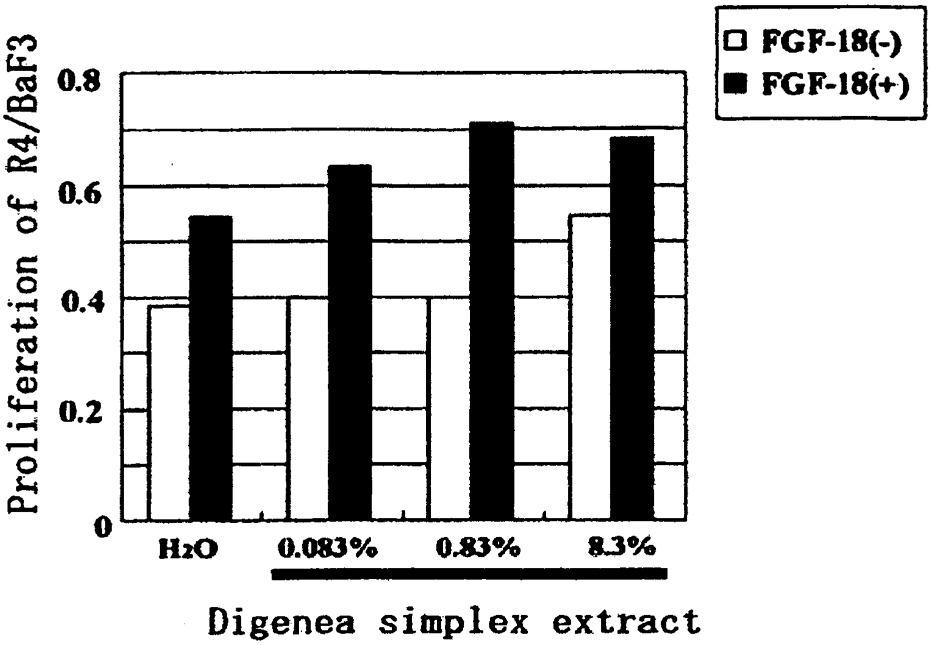
Proliferation of R4/BaF3
0.8
0.6
0.4
0.2
0
FGF-18(-)
FGF-18(+)
H2O
0.083%
0.83%
8.3%
Digenea simplex extract

Fig. 6

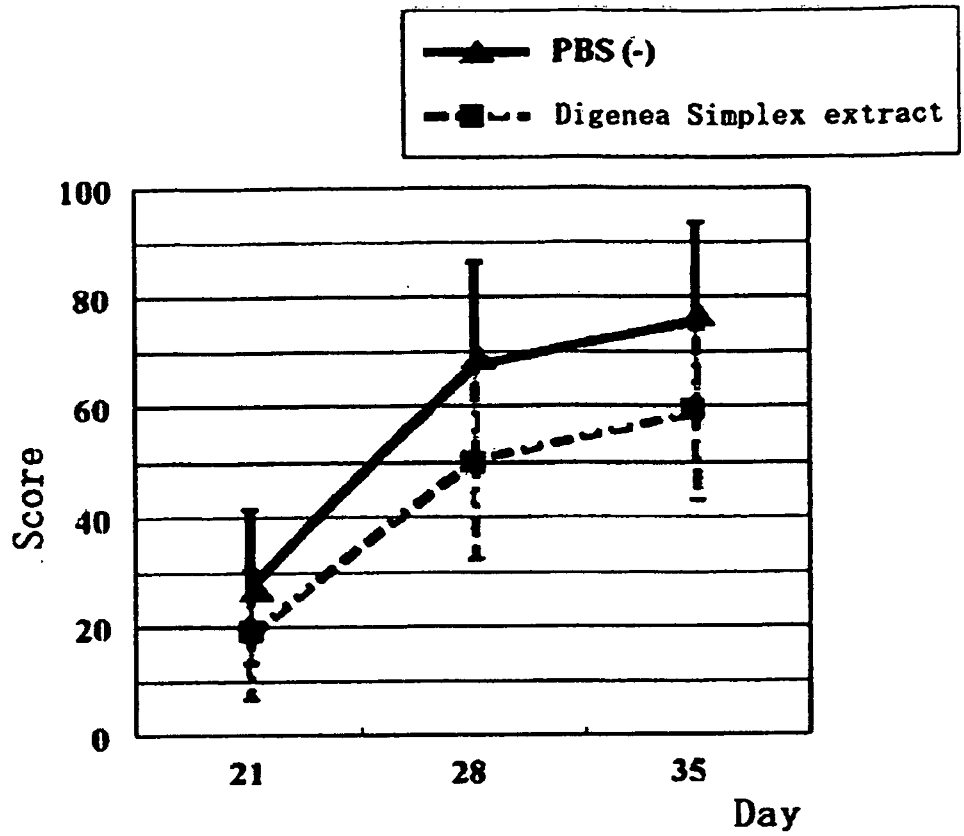

PBS (-)
Digenea Simplex extract
100
80
60
40
20
0
Score
21
28
35
Day

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP HEI4224522 B
- JP 2006083082 A
- JP 2007037844 A


### Non-patent literature cited in the description


- **ORNITZ DM ; ITOH N.** Fibroblast growth factors. *Genome Biol2: REVIE WS3005,* 2001
- **DU CROS DL.** Fibroblast growth factor and epidermal growth factor in hair development. *J Invest Dermatol,* 1993, vol. 101, 106S-113S
- **DU CROS DL ; ISAACS K ; MOORE GP.** Distribution of acidic and basicfibr oblast growth factorsin ovine skin during follicle morphogenesis. *J Cell Sci,* 1993, vol. 105, 667-674
- **HERBERT JM ; ROSENQUIST T ; GOTZ J ; MARTIN GR.** FGF5 as a regulator of the hair growth cycle: Evidence from targeted and spontaneous mutations. *Cell,* 1994, vol. 78, 1017-1025
- **DANILENKO DM ; RING BD ; YANAGIHARA D ; BENSON W ; WIEMANN B ; STAMESCO ; PIERCE GF.** Keratinocyte growth factor is an important endogenous mediator of hair follicle growth, development, and differentiation. *American J Pathol,* 1995, vol. 147, 145-154
- **MARCHESE C ; CHEDID M ; DIRSCH OR et al.** Modulation of keratinocyte growth factor and its receptor in reepithelializing human skin. *J Exp Med,* 1995, vol. 182, 1369-1376
- **GUO L ; DEGENSTEIN L ; FUCHS E.** keratinocyte growth factor is require dfor hair development but not for wound healing. *Genes Dev,* 1996, vol. 10, 165-175
- **ROSENQUIST TA ; MARTIN GR.** Fibroblast growth factor signalling in the hair growth cycle: Expression of the fibroblast growth factor receptor and ligand genes in the murine hair follicle. *Developmental Dynamics,* 1996, vol. 205, 379-386
- **PETHO-SCHRAMM A ; MULLER HJ ; PAUS R.** FGF5 and the murine hair cycle. *Arch Dermatol Res,* 1996, vol. 288, 264-266
- **MITSUI S ; OHUCHI A ; HOTTA M ; TSUBOI R ; OGAWA H.** Genes for a range of growth factors and cyclin-dependent kinase inhibitors are expressed by isolated human hair follicles. *Br J Dermatol,* 1997, vol. 137, 693-698
- **ORTEGA S ; ITTMANN M ; TSANG SH ; EHRLICH M ; BASILICO C.** Neuronaldefects and delayed wound healing in mice lacking fibroblast growth factor-2. *Proc Natl Acad Sci USA,* 1998, vol. 95, 5672-5677
- **SUZUKI S ; KATO T ; TAKIMOTO H et al.** Localization of rat FGF-5 protein in skin macrophage-like cells and FGF-5S protein in hair follicle: Possible involvement of two Fgf-5 gene products in hair growth cycleregulation. *J Invest Dermatol,* 1998, vol. 111, 963-972
- **SUZUKI S ; OTA Y ; OZAWA K ; IMAMURA T.** Dual-mode regulation of hair growth cycle by two Fgf-5 gene products. *J Invest Dermatol,* 2000, vol. 114, 456-463
- **NAKATAKE Y ; HOSHIKAWA M ; ASAKI T ; KASSAI Y ; ITOH N.** Identification of a novel fibroblast growth factor, FGF-22, preferentially expressed in the inner root sheath of the hair follicle. *Biochem Biophys Acta,* 2001, vol. 1517, 460-463
- **STENN KS ; PAUS R.** Controls of hair follicle cycling. *Physiol Rev,* 2001, vol. 81, 449-494
- **BEYER TA ; WERNER S ; DICKSON C ; GROSE R.** Fibroblast growth factor 22 and its potential role during skin development and repair. *Exp Cell Res,* 2003, vol. 287, 228-236
- **KAWANO M ; SUZUKI S ; SUZUKI M ; OKI J ; IMAMURA T.** Bulge- and basallayer-specific expression of fibroblast growth factor 13(FHF-2) in mouse skin. *J Invest Dermatol,* 2004, vol. 122, 1084-1090
- **KAWANO M ; KOMI-KURAMOCHI A ; ASADA M ; SUZUKI M ; OKI J ; JIANG J ; IMAMURA T.** Comprehensive analysis of FGF and FGFR expression in skin: FGF18 is highly expressed in hair follicles and capable of inducing anagen from telogenstage hair follicles. *J Invest Dermatol.,* 2005, vol. 124 (5), 877-885
- **ZHANGX ; IBRAHIMI OA ; OLSEN SK ; UMEMORI H ; MOHAMMADI M ; ORNITZ DM.** Receptor Specificity of the Fibroblast Growth Factor Family: THE COMPLETE MAMMALIAN FGFFAMILY. *J Biol Chem,* 2006, vol. 281 (23), 15694-15700

- *Nikkei Science,* April 1994, 20-45
- *Experimental Medicine Extra Issue,* 1994, vol. 12 (15
- Experimental Medicine Separate Volume ''Basic Techniques for Gene Therapy. Yodo-sha, 1996
- *The Pharmaceuticals Monthly,* 1994, vol. 36 (1), 23-48
- **OMITZ, DM. ; XU, J. ; COLVIN, JS. ; MCEWEN, DG ; MACARTHUR, CA. ; COULIER, F. ; GAO, G ; GOLDFARB, M.** Receptor specificity of the fibroblast growth factor family. *J. Biol. Chem.,* 1996, vol. 271 (25), 15292-15297
- **YONEDA, A. ; ASADA, M. ; ODA, Y ; SUZUKI, M ; IMAMURA, T.** Engineering of an FGF-proteoglycan fusion protein with heparin-independent, mitogenic activity. *Nat. Biotec.,* 2000, vol. 18 (6), 641-644